# EUROPEAN PATENT APPLICATION

(11) **EP 2 842 559 A2**
(43) Date of publication of application: **04.03.2015**
(21) Application number: 14185444.8
(22) Date of filing: 10.04.2006
(51) Int. Cl.: A61K 31/675, A61K 47/48, A61P 31/12

(54) **Compounds, compositions and methods for the treatment of viral infections and other medical disorders**

(30) Priority: 08.04.2005 US 669765 P
(62) Divisional of application: 06749663.8
(71) Applicant: Chimerix, Inc., Durham NC 27713 (US)
(72) Inventor: Almond, Merrick R., Apex, NC North Carolina 27502 (US); Painter, George R, Chapel Hill, NC North Carolina 27514 (US)
(74) Representative: Mintz Levin Cohn Ferris Glovsky and Popeo LLP

(57) **Abstract**

The present application provides methods and compositions for improving the bioavailability of a lipid-containing antiviral compound, and in particular, an antiviral lipid-containing compound. In one embodiment, pharmaceutically acceptable compositions are provided that include an antiviral lipid-containing compound, or salt, ester, or prodrug thereof and one or more bioavailability enhancing compounds, such as inhibitors of cytochrome P450 enzymes.

## Description

### CROSS REFERENCE TO RELATED APPLICATION

This application claims priority to U.S.S.N. 60/669,765, filed on April 8, 2005, the disclosure of which is hereby incorporated by reference in its entirety.

### TECHNICAL FIELD

This application provides a method to enhance the bioavailability, activity or other property of a lipid-containing compound such as a nucleoside or acyclic nucleoside for the treatment of a viral infection.

### BACKGROUND

Improving drug bioavailability is an established goal in the medical arts. It is important in pharmacology that a drug have sufficient bioavailability for its therapeutic purpose. The sequence of events for an oral composition includes absorption through the various mucosal surfaces, distribution via the blood stream to various tissues, biotransformation in the liver and other tissues, action at the target site, and elimination of drug or metabolites in urine or bile. Bioavailability can be reduced by poor absorption from the gastrointestinal tract, hepatic first-pass effect, or degradation of the drug prior to reaching the circulatory system.

Prodrugs are designed to be metabolized in the body (in vivo) into the active compound. Lipid prodrugs are usually designed to improve oral bioavailability, when poor absorption of the drug from the gastrointestinal tract is the limiting factor. Lipid prodrugs can also improve the selectivity of drugs to their target tissues. Lipidic molecules, including fatty acids, have been conjugated with drugs to render the conjugates more lipophilic than the unconjugated drugs. In general, increased lipophilicity has been suggested as a mechanism for enhancing intestinal uptake of drugs into the lymphatic system, thereby enhancing the entry of the conjugate into the brain and also thereby avoiding first-pass metabolism of the conjugate in the liver. The type of lipidic molecules employed have included phospholipids and fatty acids.

Phospholipid prodrugs of a number of drugs have been developed. Some of these compounds have been shown to have enhanced activity or bioavailability over that of the parent compound.

The preparation and use of alkylglycerol phosphates covalently linked to non-phosphonate containing drugs has been described (U.S. Pat. No. 5,411,947 and U.S. patent application Ser. No. 08/487,081). Prodrugs comprising alkylglycerol phosphate residues attached to antiviral nucleosides (U.S. Pat. No. 5,223,263) or phosphono-carboxylates (U.S. Pat. No. 5,463,092) have also been described. U.S. Patent No. 6,716,835 to Hostetler describes certain prodrugs of antiviral compounds, such as cidofovir. Specifically, certain derivatives of antiviral compounds, and in particular, prodrugs of cidofovir are more effective in the treatment of viruses than the parent drug. In particular, 1-0-hexadecyloxypropyl-cidofovir has enhanced efficacy over cidofovir, particularly in the treatment of pox viruses, such as smallpox.

Degradation of drugs can occur in the liver or intestine. All blood from the gastrointestinal tract passes through the liver before going elsewhere in the body in all mammals. Due to its location, liver transformation of orally dosed drugs has a substantial "first-pass effect" on drug bioavailability that was thought to exceed effects of enzyme activity in the small intestine (Tam, Y.K. "Individual Variation in First-Pass Metabolism," Clin. Pharmacokinetics 1993, 25,300-328).

Elimination of active drug by the liver occurs by one or both of two general pathways, namely biotransformation of the drug and excretion of the drug into the bile. Biotransformation reactions have been classified into two broadly defined phases. Phase I biotransformation often utilizes reactions catalyzed by the cytochrome P450 enzymes, which are manifold and active in the liver and transform many chemically diverse drugs. A second biotransformation phase can add a hydrophilic group, such as glutathione, glucuronic acid or sulfate, to increase water solubility and speed elimination through the kidneys.

Hepatocytes have contact with many types of blood and other fluid-transport vessels, such as the portal vein (nutrient and drug-rich blood from the gut), the hepatic arteries (oxygenated blood direct from the heart), the hepatic veins (efflux), lymphatics (lipids and lymphocytes), and bile ducts. The biliary ducts converge into the gall bladder and common bile duct that excretes bile into the upper intestine, aiding digestion. Bile also contains a variety of excretory products including hydrophobic drugs and drug metabolites.

It has been speculated that, in some cases, the poor bioavailability of a drug after oral administration is a result of the activity of a multidrug transporter, a membrane-bound P-glycoprotein, which functions as an energy-dependent transport or efflux pump to decrease intracellular accumulation of drug by extruding xenobiotics from the cell. It is believed that the P-glycoprotein efflux pump prevents certain pharmaceutical compounds from transversing the mucosal cells of the small intestine and, therefore, from being absorbed into the systemic circulation. A number of known non-cytotoxic pharmacological agents have been shown to inhibit P-glycoprotein, including cyclosporin, verapamil, tamoxifen, quinidine and phenothiazines, among others, (Fisher et al., Proc. Am. Soc. Clin. Oncol., 13: 143, 1994; Bartlett et al., J. Clin. Onc. 12:835-842, 1994; Lum et al., J. Clin, Onc. 10:1635-42, 1992). The administration of intravenous cyclosporin prior to or together with certain anti-cancer drugs resulted in a higher blood levels of those drugs, presumably through reduced body clearance, and exhibited the expected toxicity at substantially lower dosage levels. These findings tended to indicate that the concomitant administration of cyclosporin suppressed the MDR action of P-glycoprotein, enabling larger intracellular accumulations of the therapeutic agents. A general discussion of the pharmacologic implications for the clinical use of P-glycoprotein is provided in Lum et al., Drug Resist. Clin. Onc. Hemat., 9: 319-336 (1995); and Schinkel et al., Eur. J. Cancer, 31A: 1295-1298 (1995).

Compounds which can be administered with a drug to minimize degradation, or biotransformation, of the drug are called bioenhancers. In published PCT application WO 95/20980 (published Aug. 10, 1995) Benet et al. discloses the use of a bioenhancer comprising an inhibitor of a cytochrome P450 3A enzyme or an inhibitor of P-glycoprotein-mediated membrane transport.

There is a challenge to maximizing the effectiveness of the lipid prodrug or derivative in the body due to metabolic or other undesired actions on the *drugs in vivo.* This has been a particular problem with lipid derivatives, given the body's elaborate and complex mechanisms for degrading and synthesizing lipids.

There is a need for methods and compositions to treat viral infections with improved lipophilic compounds while minimizing the impact of drug metabolism and interactions on the therapeutic agent.

There is in particular a need for effective methods of treating orthopox virus infections in a manner which reduces the impact of drug metabolism on the therapeutic agent being administered.

There is a further need for methods for improving the bioavailability of anti-viral agents.

### SUMMARY OF THE INVENTION

The present application provides methods and compositions for improving the bioavailability of prodrugs, or a pharmaceutically acceptable salt or ester thereof, in particular, lipid-containing compounds, and in a particular embodiment, antiviral lipid-containing nucleosides. In one embodiment, a lipid containing nucleoside prodrug or other active compound is administered in combination with a bioenhancer which prevents or minimizes the metabolism or degradation of the lipid moiety. The invention may provide improved bioavailability of pharmaceutical agents, increased concentration of pharmaceutical agents in the blood, decreased dosages of drugs required for treatment of diseases and disorders and a reduction in the side effects associated with those drugs. In certain aspects, the bioenhancer is an inhibitor or substrate associated with drug biotransformation, such as one of the cytochrome P450 enzymes or an imidazole. In one embodiment, the antiviral lipid-containing compound is an anti-orthopox drug such as anti-smallpox drug. In other embodiments, the antiviral compound is active against HIV, hepatitis B, hepatitis C or other virus.

When certain prodrugs of cidofovir, such as alkoxyl alkyl phosphate esters, are administered orally, enzymes such as P450 enzymes in the liver and gut, can cause biotransformation of the prodrug, thereby reducing the efficacy of the drug. It is believed, without being limited to any theory, that the biotransformation may occur, e.g., via ω-oxidation of the terminal alkyl chain. In order to avoid the negative impact of such biotransformations, methods are provided for enhancing the bioavailability of prodrugs of antiviral compounds, in particular, nucleosides, and in particular, prodrugs of cidofovir.

A variety of bioenhancers may be used that can enhance the bioavailability of the antiviral lipid-containing compound. Enhancers can be used that reduce biotransformation of the lipid group on the compound that can occur in vivo after administration of the compound. In one embodiment, the bioavailability enhancer is an inhibitor or substrate of an enzyme associated with drug biotransformation, such as one of the cytochrome P450 enzymes, and in particular the CYP3 family of enzymes. In one embodiment, the enhancer is an imidazole (some of which have antifungal activity) for example, ketoconazole or troleandomycin; a macrolide, such as erythromycin; a calcium channel blocker, such as nifedipine; or a steroid, such as gestodene. Optionally, the compound is an inhibitor of cytochrome P450 3A (CYP3A), such as naringenin, found in grapefruit.

In one embodiment, pharmaceutically acceptable compositions are provided that include an antiviral lipid-containing compound, or salt, ester or prodrug thereof, and one or more bioavailability enhancing compounds. The compositions may be administered to a host in need thereof in an effective amount for the treatment or prophylaxis of a host infected with a virus, such as an orthopox virus.

In one embodiment, a method of treating a viral infection, e.g., an orthopox infection, is provided, the method comprising administering an effective amount of antiviral lipid-containing compound, or salt, ester or prodrug thereof, and one or more bioavailability enhancing compounds to a host in need thereof. The compositions may be administered in an effective amount for the treatment or prophylaxis of a host infected with a virus, such as an orthopox virus, optionally in combination with a pharmaceutically acceptable carrier. The compounds or compositions are administered, e.g., orally or parenterally.

In one embodiment, a method of treating a viral infection, e.g., an orthopox infection, is provided, the method comprising administering an effective amount of a prodrug of an anti-viral nucleoside containing a lipid group, or salt, ester or prodrug thereof, and one or more bioavailability enhancing compounds to a host in need thereof, wherein the bioavailability enhancer in one embodiment is an agent that reduces the degradation of the lipid group. The compositions may be administered in combination or alternation in an effective amount for the treatment or prophylaxis of a host infected with a virus, such as an orthopox virus, optionally in combination with a pharmaceutically acceptable carrier. The compounds or compositions are administered, e.g., orally or parenterally.

In one embodiment, pharmaceutical compositions are provided that may include an amount of bioavailability enhancer effective to improve the bioavailability of the antiviral lipid-containing compound in comparison to that when the compound is administered alone. In another embodiment, the enhancer is administered sequentially or together with the antiviral lipid-containing compound in an amount effective to enhance the bioavailability of the antiviral compound in comparison to that when the antiviral compound is administered without the enhancer.

In one embodiment, the antiviral compound is cidofovir, adefovir, cyclic cidofovir or tenofovir, optionally covalently linked to a lipid, or linked to an alkylglycerol, alkylpropanediol, 1-S-alkylthioglycerol, alkoxyalkanol or alkylethanediol. The enhancer is for example an imidazole antifungal, e.g., ketoconazole or troleandomycin; a macrolide, such as erythromycin; a calcium channel blocker, such as nifedipine; or a steroid, such as gestodene. Optionally, the compound is an inhibitor of cytochrome P450 3A (CYP3A), such as naringenin, found in grapefruit.

In one particular embodiment, a composition is provided that includes a cidofovir lipid prodrug and a bioavailability enhancer, such as an antifungal, wherein the composition can be administered in an effective amount for the treatment of a viral infection, such as an orthopox infection. In one embodiment, the nucleoside prodrug is an alkoxyalkyl ester of cidofovir, such as an alkoxyalkanol of cidofovir. For example, the compound may have the structure:

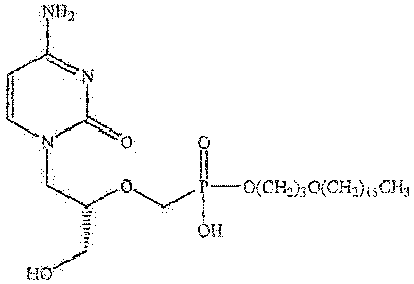

In particular, the compositions described herein can be used in methods for the prophylaxis or treatment of a host infected with a virus, in particular orthopox viruses, such as variola major and minor, vaccinia, smallpox, cowpox, camelpox, mousepox, rabbitpox, and monkeypox.

### BRIEF DESCRIPTION OF THE DRAWINGS

**Figure 1** illustrates the decrease in HDP-cidofovir over time in rabbit, Cyn monkey, Rh monkey, human, mouse and rat.
**Figure 2** illustrates the serum concentration of HDP-cidofovir, cidofovir released from HDP-cidofovir, and the metabolite M-8, which is an inactive metabolite of HDP-cidofovir, in mouse.
**Figure 3** illustrates the serum concentration of HDP-cidofovir, cidofovir released from HDP-cidofovir, and the metabolite M-8, which is an inactive metabolite of HDP-cidofovir, in NZW rabbits.
**Figure 4** illustrates the serum concentration of HDP-cidofovir, cidofovir released from HDP-cidofovir, and the metabolite M-8, which is an inactive metabolite of HDP-cidofovir, in monkey.
**Figure 5** shows a possible mechanism for the formation of M-8 by oxidation of HDP-cidofovir.
**Figure 6** shows the viral load of monkeypox titers in different types of tissue after drug administration.

### DETAILED DESCRIPTION OF THE INVENTION

Methods are provided for improving the bioavailability of a lipid containing prodrug, wherein the prodrug is administered in combination or alternation with a bioavailability enhancer. Also provided are pharmaceutically acceptable compositions comprising a lipid containing prodrug and a bioavailability enhancer. The prodrug in one embodiment is an antiviral lipid-containing compound, such as cidofovir linked to a lipid.

In one embodiment, a method of treatment of a disease or disorder is provided, the method comprising administering a lipid containing nucleoside or other active compound in combination with a bioenhancer that prevents or minimizes the metabolism or degradation of the lipid moiety. In certain aspects, the bioavailability enhancer is a compound which reduces biotransformation of the antiviral compound which can occur, for example, due to enzyme reactions with the drug, e.g., reactions with cytochrome P450 enzymes. The bioavailability enhancer is, for example, an antifungal compound or other compound that acts to reduce activity of an enzyme that is involved with biotransformation of the antiviral compound. The antiviral lipid-containing compound, is, for example, administered in an effective amount for the treatment of an orthopox infection, such as smallpox. The bioavailability enhancer is present in the composition in an effective amount to reduce the biotransformation of the drug, which can occur, for example, due to reaction with enzymes in the digestive tract or liver.

Also provided are methods for the treatment of a viral infection, such as an orthopox infection, comprising administering an effective amount of a bioavailability enhancer and an antiviral lipid-containing nucleoside.

### Prodrug Compounds

Prodrugs of a variety of compounds may be used in the methods and compositions disclosed herein. In particular, the prodrug may be one that includes a hydrocarbon chain, for example, a C4-C30, or a C8-22 hydrocarbon chain. The drug can be any of a variety of drugs, such as a variety of anticancer or antiviral compounds.

In one embodiment the prodrug is a prodrug of a nucleoside including phosphonates and phosphates. In a particular embodiment, the prodrug is antiviral lipid-containing nucleoside, such as an anti-orthopox agent.

The prodrug in one embodiment is the prodrug of an antiviral compound. The prodrug is, for example, cidofovir, adefovir, cyclic cidofovir or tenofovir, e.g., covalently linked to a lipid, such as an alkylglycerol, alkylpropanediol, 1-S-alkylthioglycerol, alkoxyalkanol or alkylethanediol, or a lipid containing a C₈-₃₀ alkyl alkenyl or alkynyl. As used herein, where a compound is "covalently linked to a lipid" the compound may include a linker between the compound and the lipid group. The lipid group is e.g., a C₈₋₃₀ alkyl, alkenyl or alkynyl.

In one embodiment, the antiviral prodrug is cidofovir, e.g., covalently linked to a lipid.

In one embodiment, the antiviral prodrug has the structure: 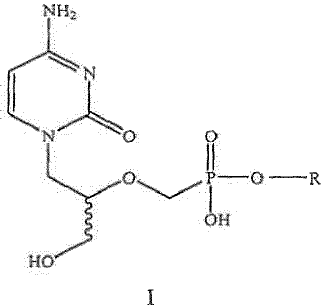 wherein R is H; optionally substituted alkyl, e.g., C₁-C₃₀ alkyl; alkenyl, e.g., C₁-C₃₀ alkenyl; or alkynyl, e,g., C₁-C₃₀ alkynyl; acyl; mono- or di-phosphate; alkylglycerol, alkylpropanediol, 1-S-alkylthioglycerol, alkoxyalkanol or alkylethanediol. In one embodiment R is an alkoxyalkanol. For example, R is -(CH₂)ₘ-O-(CH₂)ₙ-CH₃ wherein, e.g., m is 1-5 and n is 1-25; or m is 2-4 and n is 10-25.

In another embodiment, the antiviral prodrug compound has the following structure:

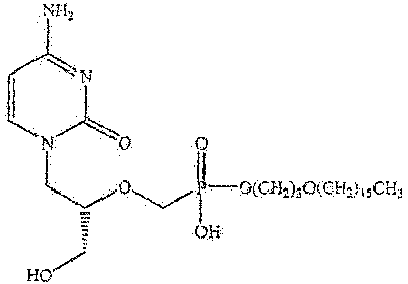

In one embodiment, the antiviral prodrug is adefovir, e.g., covalently linked to a lipid group.

In a particular embodiment, the antiviral prodrug has the following structure: 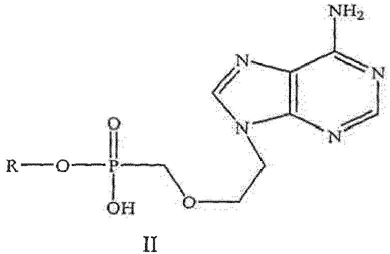 wherein R is H; an optionally substituted alkyl, e.g., C₁-C₃₀ alkyl; alkenyl, e.g., C₁-C₃₀ alkenyl; alkynyl, e.g., C₁-C₃₀ alkynyl; acyl; mono- or di-phosphate; alkylglycerol, alkylpropanediol, 1-S-alkylthioglycerol, alkoxyalkanol or alkylethanediol. In one embodiment R is an alkoxyalkanol. For example, R is -(CH₂)ₘ-O-(CH₂)ₙ-CH₃ wherein, e.g., m is 1-5 and n is 1-25; or m is 2-4 and n is 10-25.

In one embodiment, the antiviral prodrug is tenofovir, e.g., covalently linked to a lipid.

In a particular embodiment, the antiviral prodrug has the following structure: 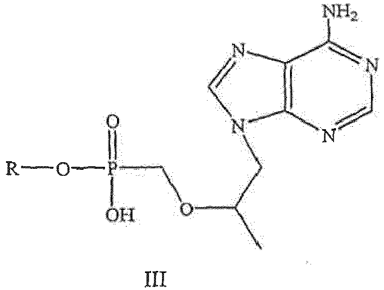 wherein R is H; an optionally substituted alkyl, e.g., C₁-C₃₀ alkyl; alkenyl, e.g., C₁-C₃₀ alkenyl; alkynyl, e.g., C₁-C₃₀ alkynyl; acyl; mono- or di-phosphate; alkylglycerol; alkylpropanediol; 1-S-alkylthioglycerol; alkoxyalkanol; or alkylethanediol. In one embodiment R is an alkoxyalkanol, R is, e.g., -(CH₂)ₘ-O-(CH₂)ₙ-CH₃ wherein, e.g., m is 1-5 and n is 1-25; or m is 2-4 and n is 10-25.

In one embodiment, the antiviral prodrug is cyclic cidofovir, e.g., covalently linked to a lipid.

In another embodiment, the antiviral prodrug has the following formula: 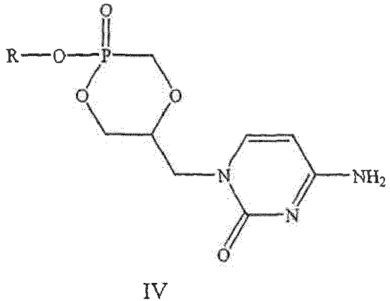 wherein R is H; an optionally substituted alkyl, e.g., C₁-C₃₀ alkyl; alkenyl, e.g., C₁-C₃₀ alkenyl; alkynyl, e.g., C₁-C₃₀ alkynyl; acyl; mono- or di-phosphate; alkylglycerol, alkylpropanediol, 1-S-alkylthioglycerol, alkoxyalkanol or alkylethanediol. In one embodiment R is an alkoxyalkanol. R is for example -(CH₂)ₘ-O-(CH₂)ₙ-CH₃ wherein, for example, m is 1-5 and n is 1-25; or m is 2-4 and n is 10-25.

In another embodiment, the prodrug is 1-0-octadecylpropanediol-3-cidofovir, 1-O-octadecylethanediol-2-cidofovir, 1-O-hexadecylpropanediol-3-cyclic cidofovir, 1-O-octadecylpropanediol-3-cyclic cidofovir, 1-O-octadecylethanediol-2-cyclic cidofovir, 1-O-hexadecylpropanediol-3-adefovir, or 1-O-octadecyl-sn-glycero-3-adefovir.

In a further embodiment, the prodrug is hexadecyloxypropyl cidofovir, octadecyloxyethyl cidofovir, oleyloxypropyl cidofovir, octyloxypropyl cidofovir, dodecyloxypropyl cidofovir, oleyloxyethyl cidofovir, 1-O-octadecyl-2-O-benzyl-glyceryl cidofovir, tetradecyloxypropyl cidofovir, eicosyl cidofovir, docosyl cidofovir, hexadecyl cidofovir, hexadecyloxypropyl cyclic cidofovir, octadecyloxyethyl cyclic cidofovir, oleyloxypropyl cyclic cidofovir, octyloxypropyl cyclic cidofovir, dodecyloxypropyl cyclic cidofovir, oleyloxyethyl cyclic cidofovir, 1-O-octadecyl-2-O-benzyl-glyceryl cyclic cidofovir, tetradecyloxypropyl cyclic cidofovir, eicosyl cyclic cidofovir, docosyl cyclic cidofovir, or hexadecyl cyclic cidofovir.

Compounds described in U.S. Patent No. 6,716,825, the disclosure of which is incorporated herein, can be used in the methods and compositions provided herein. The compounds may be made by methods available in the art, such as those described in U.S. Patent No. 6,716,825.

A variety of lipid derivatives of antiviral compounds, e.g., antiviral nucleosides, can be used in the methods and compositions provided herein. In one embodiment, the antiviral compounds are in prodrug form and have one of the following structures: 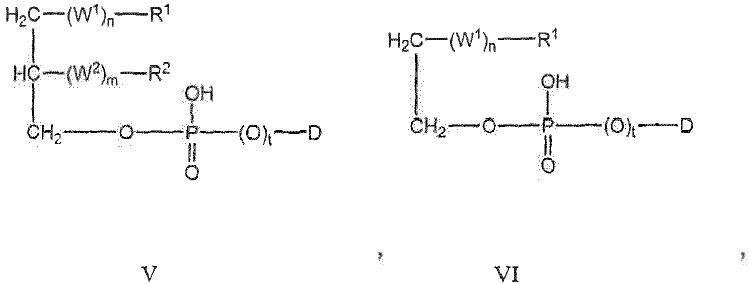 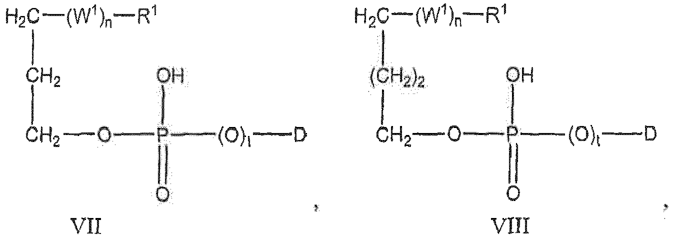 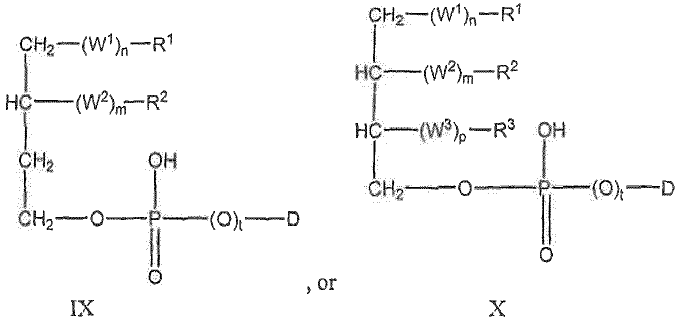 wherein W¹, W², and W³ are each independently -O-, -S-, -SO-, -SO₂, -O(C=O)-, -(C=O)O-, -NH(C=O)-, -(C=O)NH- or -NH-; and in one embodiment are each independently O, S, or -O(C=O)-;
n is 0 or 1; m is 0 or 1; p is 0 or 1
R¹ is an optionally substituted alkyl, alkenyl or alkynyl, e.g., C_{1 -30} alkyl, alkenyl, or alkynyl; or in one embodiment, R¹ is optionally C₈₋₃₀ alkyl, alkenyl or alkynyl, or R¹ is a C₈₋₂₄ alkyl, alkenyl or alkynyl (e.g., C₁₇) C₁₈) C₁₉, C₂₀, C₂₁, C₂₂, C₂₃, or C₂₄ alkyl, alkenyl, or alkynyl);
R² and R³ are each independently an optionally substituted C₁₋₂₅ alkyl, alkenyl, or alkynyl; or in one embodiment, optionally R² and R³ are each independently C₁₋₅ alkyl, alkenyl, or alkynyl (e.g., C₁, C₂, or C₃ alkyl, alkenyl or alkynyl; e.g., methyl, ethyl or propyl); or in another embodiment CF₃; or in another embodiment aryl, e.g., benzyl;
t is 0 or 1; and
D is an antiviral drug, e.g., an antiviral cyclic or acyclic nucleoside.

In one subembodiment of the formulas disclosed herein, including Formulas V-X, t is 1 and 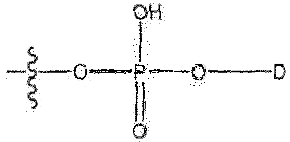 is a residue of a biologically active phosphate drug, including but not limited to a 5'-O-phosphate nucleoside, 2'-O-phosphate nucleoside, or 3'-O-phosphate nucleoside.

In another subembodiment of the formulas disclosed herein, including Formulas V-X, t is 0 and 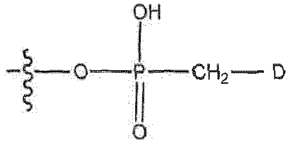 is a residue of a biologically active phosphonate drug, including but not limited to cidofovir, adefovir, tenofovir, cyclic cidofovir, HPMPA, PMEG, or any other phosphonate derivative of a biologically active nucleoside or acyclic nucleoside.

In another embodiment, the prodrug is a 1-O-alkyl-propanediol-phosphate of an antiviral nucleoside, wherein the antiviral nucleoside has (1) a substituted or unsubstituted purine or pyrimidine with either (a) an acyclic hydroxylated fragment of a ribose residue, (e.g., a hydroxylated 2-propoxymethyl or ethoxymethyl) (b) a ribose or 2'-deoxyribose, or (c) deoxypentose.

In another embodiment, the prodrug is a 1-O-alkyl-ethanediol-phosphate of an antiviral nucleoside, wherein the antiviral nucleoside has (1) a substituted or unsubstituted purine or pyrimidine with either (a) an acyclic hydroxylated fragment of a ribose residue, (e.g., a hydroxylated 2-propoxymethyl or ethoxymethyl) (b) a ribose or 2'-deoxyribose, or (c) deoxypentose.

In one subembodiment of Formulas V-X:
W¹, W², and W³ are each independently -O-, -S-, or -O(CO)-;
n is 0 or 1; m is 0 or 1; p is 0 or 1
R¹ is an optionally substituted C₁₈₋₂₄ alkyl or alkenyl (e.g., C₁₈, C₁₉, C₂₀, C₂₁, C₂₂, C₂₃, or C₂₄ alkyl);
R² and R³ are each independently an optionally substituted C₁₋₅ alkyl, alkenyl, alkynyl or CF₃; (e.g., C₁, C₂, or C₃ alkyl; e.g., methyl or ethyl) or cycloalkyl; t is 0 or 1; and
D is an antiviral cyclic or acyclic nucleoside.

In another subembodiment, the antiviral prodrug is one of the following structures: 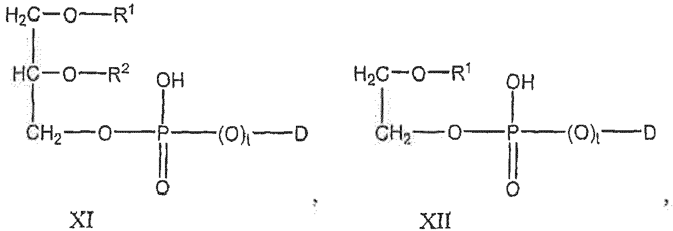 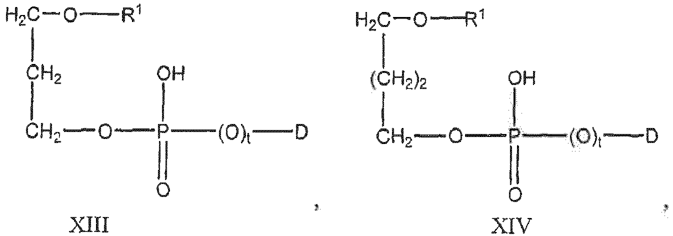 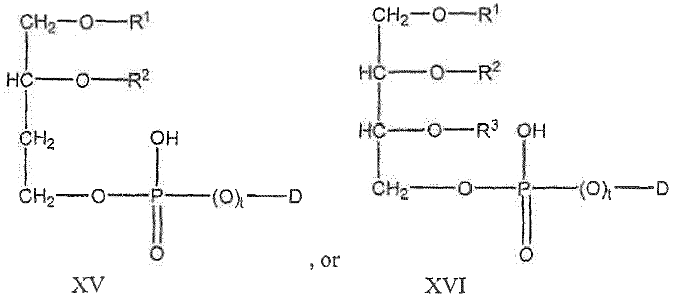 wherein R¹ is an optionally substituted C₈₋₂₄ alkyl, for example, C₁₈₋₂₄ alkyl (e.g., C₁₈, C₁₉, C₂₀, C₂₁, C₂₂, C₂₃, or C₂₄ alkyl);
R² and R³ are independently C₁₋₅ alkyl, haloalkyl, alkenyl, alkynyl, or cycloalkyl e.g. methyl, ethyl or CF₃;
t is 0 or 1; and
D is an antiviral drug, e.g., an antiviral cyclic or acyclic nucleoside.

Exemplary antiviral nucleosides that can be linked to lipid groups, for example, as shown in Formulas V-XVI described above, are ddA, ddI, ddG, L-FMAU, DXG, DAPD, L-dA, L-dI, L-(d)T, L-dC, L-dG, FTC, 5-FC, 1-(2'-deoxy-2'-fluoro-1-β-D-arabinofuranosyl)-5-iodocytosine (FIAC) or 1(2'-deoxy-2'-fluoro-1-β-D-arabinofuranosyl)-5-iodouracil (FIAU) and the like.

Other nucleosides (e.g., useful in treating poxvirus infections) that can be used optionally covalently derivatized to include a lipid group, e.g. as illustrated in Formulas V-XVI include 8-methyladenosine, 2-amino-7-[(1,3-dihydroxy-2-propoxy)methyl]purine (S2242), Ara-A, PME-N6-(cyclopropyl) DAP, phosphonomethoxyethyl deoxydiaminopurine (PMEADADP); PME-N6-(dimethyl)DAP, PME-N6-(trifluoroethyl)DAP, PMEA-N6-(2-propenyl)DAP, analogs of adenosine-N(1)-oxide, analogs of 1-(benzyloxy) adenosine, IMP dehydrogenase inhibitors (e.g., ribavirin, EICAR, tiazofurin, and selenazole), SAH hydrolase inhibitors (e.g., 5'-noraristeromycin, neplanocins A and C, carbocyclic 3-deaza-adenosine, DHCeA, C³DHCeA, 6'-β-fluoro-aristeromycin, 5'-noraristeromycin and epimers thereof, 3-deaza-5'-noraristeromycin, 6'-C-methylneplanocin, 6'-homoneplanocin, 2-fluoroneplanocin, 6'-iodo acetylenic Ado, and 3-deazaneplanocin), OMP decarboxylase inhibitors (e.g., pyrazofurin and 5'-deoxypyrazofurin) CTP synthetase inhibitors (e.g., cyclopentenyl cytosine and carbodine), thymidylate sythase inhibitors (e.g., 5-substituted 2'-deoxyuridines), and, 3'-fluoro-3'-deoxyadenosine.

Other nucleosides include 3'-azido-2',3'-dideoxypyrimidine nucleosides, for example, AZT, AZT-P-AZT, AZT-P-ddA, AZT-P-ddI, AzddClU, AzddMeC, AzddMeC N4-OH, AzddMeC N4Me, AZT-P-CyE-ddA, AzddEtU(CS-85), AzddU(CS-87), AzddC(CS-91), AzddFC, AzddBrU, and AzddIU; the class comprising 3'-halopyrimidine dideoxynucleosides, for example, 3'-FddClU, 3'-FddU, 3'-FddT, 3'-FddBrU, and 3'-FddEtU; the class comprising 2',3'-didehydro-2',3'-dideoxynucleosides (D4 nucleosides), for example, D4T, D4C, D4MeC, and D4A; the class comprising 2',3'-unsubstituted dideoxypyrimidine nucleosides, for example, 5-F-ddC, ddC and ddT; the class comprising 2',3'-unsubstituted dideoxypurines nucleosides, for example, ddA, ddDAPR(diaminopurine), ddG, ddI, and ddMeA(N6 methyl); and the class comprising sugar-substituted dideoxypurine nucleosides, for example, 3-N₃ ddDAPR, 3-N₃ ddG, 3-FddDAPR, 3-FddG, 3-FddaraA, and 3-FddA, wherein Me is methyl, Et is ethyl and CyEt is cyanoethyl.

Other exemplary nucleosides include the didehydropyrimidines, as well as carbovir, a carbocyclic 2',3'- didehydroguanosine; the 3'-azido derivatives of deoxyguanosine (AZG) and the pyrimidine, deoxyuridine; the 3'-fluoro derivatives of deoxythymidine and deoxyguanosine; the 2',6'-diaminopurines, 2',3'-deoxyriboside and its 3'-fluoro and 3'-azido derivatives; 2-chloro-deoxyadenosine; ganciclovir, acyclovir, cyclic ganciclovir, 9-(2-phosphonylmethoxyethyl)guanine (PMEG), 9-(2 phosphonyl-methoxyethyl)adenine (PMEA), penciclovir, cidofovir, adefovir, cyclic cidofovir, 9-(3-hydroxy-2-phosphonylmethoxypropyl)adenine (HPMPA), cHPMPA, 8-Aza-HPMPA, HPMPG, (S)-9-(3-hydroxy-2-phosphonylmethoxypropyl)-2,6-diaminopurine ((S)-HPMPDAP), (S)-6-(3-hydroxy-2-phosphonylmethoxypropyl)oxy-2,4-diaminopyrimidine ((S)-HPMPO-DAPy), and tenofovir.

Many phosphonate compounds can be derivatized into lipid containing compounds to improve their pharmacologic activity, or to increase their oral absorption, such as, for example, the compounds disclosed in the following patents, each of which are hereby incorporated by reference in their entirety: U.S. Pat. No. 3,468,935 (Etidronate), U.S. Pat. No. 4,327,039 (Pamidronate), U.S. Pat. No. 4,705,651 (Alendronate), U.S. Pat. No. 4,870,063 (Bisphosphonic acid derivatives), U.S. Pat. No. 4,927,814 (Diphosphonates), U.S. Pat. No. 5,043,437 (Phosphonates of azidodideoxynucleosides), U.S. Pat. No. 5,047,533 (Acyclic purine phosphonate nucleotide analogs), U.S. Pat. No. 5,142,051 (N-Phosphonylmethoxyalkyl derivatives of pyrimidine and purine bases), U.S. Pat. No. 5,183,815 (Bone acting agents), U.S. Pat. No. 5,196,409 (Bisphosphonates), U.S. Pat. No. 5,247,085 (Antiviral purine compounds), U.S. Pat. No. 5,300,671 (Gem-diphosphonic acids), U.S. Pat. No. 5,300,687 (Trifluoromethylbenzylphosphonates), U.S. Pat. No. 5,312,954 (Bis- and tetrakis-phosphonates), U.S. Pat. No. 5,395,826 (Guanidinealkyl-1,1-bisphosphonic acid derivatives), U.S. Pat. No. 5,428,181 (Bisphosphonate derivatives), U.S. Pat. No. 5,442,101 (Methylenebisphosphonic acid derivatives), U.S. Pat. No. 5,532,226 (Trifluoromethybenzylphosphonates), U.S. Pat. No. 5,656,745 (Nucleotide analogs), U.S. Pat. No. 5,672,697 (Nucleoside-5'-methylene phosphonates), U.S. Pat. No. 5,717,095 (Nucleotide analogs), U.S. Pat. No. 5,760,013 (Thymidylate analogs), U.S. Pat. No. 5,795,340 (Nucleotide analogs), U.S. Pat. No. 5,840,716 (Phosphonate nucleotide compounds), U.S. Pat. No. 5,856,314 (Thio-substituted, nitrogen-containing, heterocyclic phosphonate compounds), U.S. Pat. No. 5,885,973 (olpadronate), U.S. Pat. No. 5,886,179 (Nucleotide analogs), U.S. Pat. No. 5,877,166 (Enantiomerically pure 2-aminopurine phosphonate nucleotide analogs), U.S. Pat. No. 5,922,695 (Antiviral phosphonomethoxy nucleotide analogs), U.S. Pat. No. 5,922,696 (Ethylenic and allenic phosphonate derivatives of purines), U.S. Pat. No. 5,977,089 (Antiviral phosphonomethoxy nucleotide analogs), U.S. Pat. No. 6,043,230 (Antiviral phosphonomethoxy nucleotide analogs), U.S. Pat. No. 6,069,249 (Antiviral phosphonomethoxy nucleotide analogs); Belgium Patent No. 672205 (Clodronate); European Patent No. 753523 (Amino-substituted bisphosphonic acids); European Patent Application 186405 (geminal diphosphonates); and the like. In addition, the compounds listed in the following publications can be derivatized to improve their pharmacologic activity, or to increase their oral absorption; each of which are hereby incorporated herein by reference in their entirety: J. Med. Chem., 2002, 45:1918-1929; J. Med. Chem., 2003, 46:5064-5073; Antimicrob. Agents Chemotherapy, 2002, 46:2185-2193.

Nucleosides can be derivatized with a variety of lipophilic groups as described in the following patents and can be used in the compositions and methods provided herein: U.S. Patent No. 5,614,548; U.S. Patent No. 5,512,671; U.S. Patent No. 5,770,584, U.S. Patent No. 5,962,437; U.S. Patent No. 6,030,960; U.S. Patent No. 6,670,341; U.S. Patent No. 5,223,263; U.S. Patent No. 5,817,638; U.S. Patent No. 6,252,060; U.S. Patent No. 6,448,392; U.S. Patent No. 5,411,947; U.S. Patent No. 5,744,592; U.S. Patent No. 5,484,809; U.S. Patent No. 5,827,831; U.S. Patent No. 5,696,277; U.S. Patent No. 6,002,029; U.S. Patent No. 5,780,617; U.S. Patent No. 5,194,654; U.S. Patent No. 5,463,092; U.S. Patent No. 5,744,461; U.S. Patent No. 5,484,911; WO 91/09602; WO 91/05558; U.S. Patent No. 4,444,766; U.S. Pat. No. 5,869,468; U.S. Patent No. 5,84,228; U.S. Publication No. 2002/0082242; U.S. Publication No. 2004/0161398; U.S. Publication No. 2004/0259845; WO 98/38202; U.S. Patent No. 5,696,277; U.S. Patent No. 6,002,029; U.S. Patent No. 5,744,592; U.S. Patent No. 5,827,831; U.S. Patent No. 5,817,638; and U.S. Patent No. 6,252,060 and U.S. Patent No. 5,756,711, each of which are incorporated herein by reference in their entirety.

Prodrugs of other compounds also may be used including prodrugs of the following agents: analgesic; anesthetic; anorectic; anti-adrenergic; anti-allergic; anti-anginal; anti-anxiety; anti-arthritic; anti-asthmatic; anti-atherosclerotic; antibacterial; anticoagulant; anticonvulsant; antidepressant; antidiabetic; antidiarrheal; antidiuretic; anti-estrogen; antifibrinolytic; antifungal; antiglaucoma agent; antihistamine; anti-infective; anti-inflammatory; antikeratinizing agent; antimalarial; antimicrobial; antimigraine; antimitotic; antimycotic, antinauseant, antineoplastic, antineutropenic, antiobessional agent; antiparasitic; antiparkinsonian; antiperistaltic, antipneumocystic; antiproliferative; liver disorder treatment;psychotropic; serotonin inhibitor; serotonin receptor antagonist; steroid; stimulant; suppressant; thyroid hormone; thyroid inhibitor; thyromimetic; tranquilizer; agent for treatment of amyotrophic lateral sclerosis; agent for treatment of cerebral ischemia; agent for treatment of Paget's disease; agent for treatment of unstable angina; uricosuric; vasoconstrictor; vasodilator; vulnerary; or a wound healing agent.

Prodrugs of the following anticancer agents that can be used include prodrugs of Antineoplastic agents such as: Acivicin; Aclarubicin; Acodazole Hydrochloride; Acronine; Adozelesin; Adriamycin; Aldesleukin; Alitretinoin; Allopurinol Sodium; Altretamine; Ambomycin; Ametantrone Acetate; Aminoglutethimide; Amsacrine; Anastrozole; Annonaceous Acetogenins; Anthramycin; Asimicin; Asparaginase; Asperlin; Azacitidine; Azetepa; Azotomycin; Batimastat; Benzodepa; Bexarotene; Bicalutamide; Bisantrene Hydrochloride; Bisnafide Dimnesylate; Bizelesin; Bleomycin Sulfate; Brequinar Sodium; Bropirimine; Bullatacin; Busulfan; Cabergoline; Cactinomycin; Calusterone; Caracemide; Carbetimer; Carboplatin; Carmustine; Carubicin Hydrochloride; Carzelesin; Cedefingol; Celecoxib; Chlorambucil; Cirolemycin; Cisplatin; Cladribine; Crisnatol Mesylate; Cyclophosphamide; Cytarabine; Dacarbazine; DACA (N-[2-(Pimethyl-amino)ethyl]acridine-4-carboxamide); Dactinomycin; Daunorubicin Hydrochloride; Daunomycin; Decitabine; Denileukin Diftitox; Dexormaplatin; Dezaguanine; Dezaguanine Mesylate; Diaziquone; Docetaxel; Doxorubicin; Doxorubicin Hydrochloride; Droloxifene; Droloxifene Citrate; Dromostanolone Propionate; Duazomycin; Edatrexate; Eflornithine Hydrochloride; Elsamitrucin; Enloplatin; Enpromate; Epipropidine; Epirubicin Hydrochloride; Erbulozole; Esorubicin Hydrochloride; Estramustine; Estramustine Phosphate Sodium; Etanidazole; Ethiodized Oil I 131; Etoposide; Etoposide Phosphate; Etoprine; Fadrozole Hydrochloride; Fazarabine; Fenretinide; Floxuridine; Fludarabine Phosphate; Fluorouracil; 5-FdUMP; Flurocitabine; Fosquidone; Fostriecin Sodium; FK-317; FK-973; FR-66979; FR-900482; Gemcitabine; Gemcitabine Hydrochloride; Gemtuzumab Ozogamicin; Gold Au 198; Goserelin Acetate; Guanacone; Hydroxyurea; Idarubicin Hydrochloride; Ifosfamide; Ilmofosine; Interferon Alfa-2a; Interferon Alfa-2b; Interferon Alfa-n1; Interferon Alfa-n3; Interferon Beta-I a; Interferon Gamma-I b; Iproplatin; Irinotecan Hydrochloride; Lanreotide Acetate; Letrozole; Leuprolide Acetate; Liarozole Hydrochloride; Lometrexol Sodium; Lomustine; Losoxantrone Hydrochloride; Masoprocol; Maytansine; Mechlorethamine Hydrochloride; Megestrol Acetate; Melengestrol Acetate; Melphalan; Menogaril; Mercaptopurine; Methotrexate; Methotrexate Sodium; Methoxsalen; Metoprine; Meturedepa; Mitindomide; Mitocarcin; Mitocromin; Mitogillin; Mitomalcin; Mitomycin; Mytomycin C; Mitosper; Mitotane; Mitoxantrone Hydrochloride; Mycophenolic Acid; Nocodazole; Nogalamycin; Oprelvekin; Ormaplatin; Oxisuran; Paclitaxel; Pamidronate Disodium; Pegaspargase; Peliomycin; Pentamustine; Peplomycin Sulfate; Perfosfamide; Pipobroman; Piposulfan; Piroxantrone Hydrochloride; Plicamycin; Plomestane; Porfimer Sodium; Porfiromycin; Prednimustine; Procarbazine Hydrochloride; Puromycin; Puromycin Hydrochloride; Pyrazofurin; Riboprine; Rituximab; Rogletimide; Rolliniastatin; Safingol; Safingol Hydrochloride; Samarium/Lexidronam; Semustine; 7-hydroxystaurosporine; Simtrazene; Sparfosate Sodium; Sparsomycin; Spirogermanium Hydrochloride; Spiromustine; Spiroplatin; Squamocin; Squamotacin; Streptonigrin; Streptozocin; Strontium Chloride Sr 89; Sulofenur; Talisomycin; Taxane; Taxoid; Tecogalan Sodium; Tegafur; Teloxantrone Hydrochloride; Temoporfin; Teniposide; Teroxirone; Testolactone; Thiamiprine; Thioguanine; Thiotepa; Thymitaq; Tiazofurin; Tirapazamine; Tomudex; TOP-53; Topotecan Hydrochloride; Toremifene Citrate; Trastuzumab; Trestolone Acetate; Triciribine Phosphate; Trimetrexate; Trimetrexate Glucuronate; Triptorelin; Tubulozole Hydrochloride; Uracil Mustard; Uredepa; Valrubicin; Vapreotide; Verteporfin; Vinblastine; Vinblastine Sulfate; Vincristine; Vincristine Sulfate; Vindesine; Vindesine Sulfate; Vinepidine Sulfate; Vinglycinate Sulfate; Vinleurosine Sulfate; Vinorelbine Tartrate; Vinrosidine Sulfate; Vinzolidine Sulfate; Vorozole; Zeniplatin; Zinostatin; Zorubicin Hydrochloride; 2-Chlorodeoxyadenosine; 2'-Deoxyformycin; 9-aminocamptothecin; raltitrexed; N-propargyl-5,8-dideazafolic acid; 2-chloro-2'-arabino-fluoro-2'-deoxyade- nosine; 2-chloro-2'-deoxyadenosine; anisomycin; trichostatin A; hPRL-G129R; CEP-751; linomide; sulfur mustard; nitrogen mustard (mechlor ethamine); cyclophosphamide; melphalan; chlorambucil; ifosfamide; busulfan; N-methyl-N-nitrosourea (MNU); N, N'-Bis(2-chloroethyl)-N-nitros- ourea (BCNU); N-(2-chloroethyl)-N'-cyclohexyl-N-nitrosourea (CCNU); N-(2-chloroethyl)-N'-(trans-4-methylcyclohexyl-N-nitrosourea (MeCCNU); N-(2-chloroethyl)-N'-(diethyl)ethylphosphonate-N-nitrosourea (fotemustine); streptozotocin; diacarbazine (DTIC); mitozolomide; temozolomide; thiotepa; mitomycin C; AZQ; adozelesin; Cisplatin; Carboplatin; Ormaplatin; Oxaliplatin; C1-973; DWA 2114R; JM216; JM335; Bis (platinum); tomudex; azacitidine; cytarabine; gemcitabine; 6-Mercaptopurine; 6-Thioguanine; Hypoxanthine; teniposide; 9-amino camptothecin; Topotecan; CPT-11; Doxorubicin; Daunomycin; Epirubicin; darubicin; mitoxantrone; losoxantrone; Dactinomycin (Actinomycin D); amsacrine; pyrazoloacridine; all-trans retinol; 14-hydroxy-retro-retinol; all-trans retinoic acid; N-(4-Hydroxyphenyl) retinamide; 13-cis retinoic acid; 3-Methyl TTNEB; 9-cis retinoic acid; fludarabine (2-F-ara-AMP); and 2-chlor odeoxyadenosine (2-Cda).

### Enhancers

A variety of bioavailability enhancing agents may be administered or may be present in a pharmaceutical composition in an amount effective to enhance the bioavailability of the lipid containing prodrug, such as an antiviral lipid-containing nucleoside. The bioavailability enhancer is used to minimize degradation, or biotransformation, of the drug. In one embodiment, the bioavailability enhancer prevents or minimizes the metabolism or degradation of the lipid moiety of a lipid prodrug.

"Drug bioavailability" refers to total amount of drug systemically available over time. Drug bioavailability can be increased by inhibiting drug biotransformation in the gut and/or by inhibiting active transport systems in the gut which decrease the net transport of drugs across gut epithelia, and/or by decreasing drug biotransformation in the liver. The compound causing increased drug bioavailability of the prodrug is referred to herein as a bioenhancer or bioavailability enhancer.

Any bioassay that determines whether a given compound has the inhibition or binding characteristics required of a bioenhancer can be used to identify compounds that can be used.

In one embodiment, the bioavailability enhancer is an inhibitor or substrate of an enzyme associated with drug biotransformation, such as one of the cytochrome P450 enzymes. In one embodiment, the enhancer is an antifungal, such as an imidazole antifungal, e.g., ketoconazole or troleandomycin; a macrolide, such as erythromycin; a calcium channel blocker, such as nifedipine; or a steroids, such as gestodene. Optionally, the compound is an inhibitor of cytochrome P450 3A (CYP3A), such as naringenin, found in grapefruit.

Any agent that will effect the activity of an enzyme or receptor involved in drug biotransformation may be used. In one embodiment, the enzymes for which activity can be reduced are cytochrome P450 enzymes, and in particular the CYP3 family of enzymes.

The cytochromes P450 are a superfamily of hemoproteins. They represent the terminal oxidases of the mixed function oxidase system. The cytochrome P450 gene superfamily is composed of at least 207 genes that have been named based on the evolutionary relationships of the cytochromes P450. For this nomenclature system, the sequences of all of the cytochrome P450 genes are compared, and those cytochromes P450 that share at least 40% identity are defined as a family (designated by ***CYP*** followed by a Roman or Arabic numeral, e.g. CYP3), further divided into subfamilies (designated by a capital letter, e.g. CYP3A), which are comprised of those forms that are at least 55% related by their deduced amino acid sequences. Finally, the gene for each individual form of cytochrome P450 is assigned an Arabic number (e.g. CYP3A4).

Three cytochrome P450 gene families (CYP1, CYP2 and CYP3) appear to be responsible for most drug metabolism. At least 15 cytochromes P450 have been characterized to varying degrees in the human liver. The CYP3 gene family encoding cytochromes P450 of type 3 is possibly the most important family in human drug metabolism. At least 5 forms of cytochrome P450 are found in the human 3A subfamily, and these forms are responsible for the metabolism of a large number of structurally diverse drugs. The liver contains many isoforms of cytochrome P450 and can biotransform a large variety of substances. The enterocytes lining the lumen of the intestine also have significant cytochrome P450 activity, and this activity is dominated by a single family of isozymes, 3A, the most important isoforms in drug metabolism. Thus, in one embodiment, drug efficacy is increased by reducing CYP3a drug biotransformation, for example, in the liver and/or intestinal lumen.

In particular, the activity of the cytochrome P450 enzymes can be inhibited or the cytochrome P450 enzymes can be inactivated by drugs and environmental compounds. This includes competitive inhibition between substrates of the same cytochrome P450, inhibition by agents that bind sites on the cytochrome P450 other than the active site, and suicidal inactivation of the cytochrome P450 by reactive intermediates formed during the metabolism of an agent. For example, inhibitors can function by acting as a competitive, non-competitive, uncompetitive, mixed or irreversible inhibitor of CYP3A drug biotransformation. The inhibitor may also act by binding to the drug being protected, either by covalent bonding or by ionic or polar attractions.

The activity of CYP3A is CYP3A catalyzed production of reaction product from CYP3A substrates. Substrates for CYP3A can be naturally occurring substrates or other components such as those listed in Table 1. In addition, some of the CYP3A inhibitors listed in Table 1 have been identified as substrates, as designated in the table. The catalytic activities of CYP3A, subject to inhibition, include dealkyase, oxidase, and hydrolase activities. In addition to the different catalytic activities of CYP3A, different forms of CYP3A exist with a range in molecular weight (for example, from 51 kD to 54 kD, as shown in Komori et al., J. Biochem. 1988, 104:912-16). The compounds listed in Table 1, in particular the inhibitors, can be used as enhancers in the methods and compositions described herein.

**TABLE 1**

| P450 3A substrates | P450 3A inhibitors |
|---|---|
| Antiarrhythmic | Antidiabetic |
| Amiodarone | Glibenclamide |
| Lidocaine | Tolbutamide |
| Quinidine | Benzodiazepine |
| Antiepileptic | Midazolam* |
| Etnosuximide | Calcium channel blocker |
| Zonisamide | Diluazem |
| Antidepressant | Felodipine |
| Imipramine | Nicardipine |
| Tianeptine | Nifedipine* |
| Benzodiazepine | Verapamil |
| Clonazepam | Chemotherapeutic |
| Diazepam | Clotrimazole |
| Triazolam | Erythromycin* |
| Chemotherapeutics | Fluconazole |
| Dapsone | Itraconazole |
| Ifosfamide | Josamycin |
| Environmental toxins | Ketoconazole |
| 1.6-dinitropyrene | Miconazole |
| 1-nitropyrene | Midecamycin |
| 6-nitrochrysene | Navelbine* |
| Aflatoxin B1 | Primaquine |
| Benzo(a)pyrene | Triacetylotendomycin* |
| MOCA.sup.1 | Vinblastine* |
| PhIP.sup.2 | Vincristine* |
| Immunosuppressant | Vindesine* |
| Cyclosporine | Flavanoids |
| FK-506 | Benzonavone |
| Rapamycin | Kaempferol |
| Narcotic | Naringenin |
| Alfentanil | Quercetin |
| Cocaine | Steroid hormone |
| Codeine | Cortisol* |
| Ethyhmorphine | Ethinylestradiol* |
| Steroid hormones | Gestodene |
| 17αethynylestradiol | Methylprednisolone |
| Estradiol | Norgestrel |
| Flutamide | Prednisolone |
| Testosterone | Prednisone |
| Miscellaneous | Progesterone* |
| 1-tetrahydrocannabinol | Tamoxifen* |
| Acetaminophen | Thiotestosterone |
| Benzphetamine | Miscellaneous |
| Dextromethorphan | Bromocriptine |
| Digitoxin | DDEP |
| Lovastatin | Dihydroergotamine |
| NOHA³ | Ergotamine |
| Retinoic acid | |
| Selegiline | |
| Terfenadine | |

| | |
|---|---|
| *Drugs marked * have also been identified as P450 3A substrates 1 MOCHA: 4,4'-Methylenebis(2-Chloroaniline) 2 PhIP: 2amino-1-methyl-6-phenylimidazo[4,5-b]pyridine 3 NOHA: Nomega-hydroxy-L-arginine 4 DDEP: 3,5dicarbetoxy-2,6-dimethyl-4-ethyl-1,4-dihydropyridine | |

In a particular embodiment, the enhancer is an inhibitor of CYA enzymes such as paroxetine, fluoxetine, sertreline, fluvoxamine, nefazodone, venlafaxine, cimetidine, fluphenazine, haloperidol, perphenazine, thioridazine, diltiazem, metronidazole, troleandomyan, disulfiram, St. John's Wort, and omeprazole.

Enhancers also include compounds that inhibit P-glycoprotein, such as cyclosporin, verapamil, tamoxifen, quinidine and phenothiazines.

Exemplary enhancers include anti-viral protease inhibitors, e.g., indinavir, nelfinavir, ritonavir, saquinavir; and anti-fungal agents, e.g., fluconazole, itraconazole, ketoconazole, and miconazole.

Other enhancers include macrolides such as clarithromycin, erythromycin, nortriptyline, lignocaine, and anriodarone.

Other enhancers include 17-ethinyl-substituted steroids, for example, gestodene, ethinyl-estradiol, methoxsalen, and levonorgestrol.

Other enhancers include flavones such as quercetin and naringenin, and other compounds such as ethynyl estradiol, and prednisolone.

In one embodiment, the bioavailability enhancer is an inhibitor of P-glycoprotein (P-gp)-mediated membrane transport.

In another embodiment, the bioavailability enhancer is cyclosporine A, active blockers GF120918 (elacridar), LY335989 (zosuquidar), valspodar (PSC833), biricodar (VX 710), or R101933.

Tests for active enhancers that are available in the art may be used to select the appropriate compounds. For example, enzyme inhibition may be measured. In one embodiment, cultured cells of hepatocytes or enterocytes or freshly prepared cells from either liver or gut can be used to determine the ability of a compound to act as a CYP3A inhibitor. Various methods of gut epithelial cell isolation can be used such as the method of Watkins et al., J. Clin. Invest. 1985; 80:1029-36. Cultured cells, as described in Schmiedlin-Ren, P. et al., Biochem. Pharmacol. 1993; 46:905-918, can also be used. The production of CYP3A metabolites in cells can be measured using high pressure liquid chromatograph (HPLC) methods as described in the following section for microsome assays of CYP3A activity.

Microsomes from hepatocytes or enterocytes can also be used for CYP3A assays. Microsomes can be prepared from liver using conventional methods as discussed in Kronbach et al., Clin. Pharmacol. Ther 1988; 43:630-5. Alternatively, microsomes can be prepared from isolated enterocytes using the method of Watkins et al., J. Clin. Invest. 1987; 80:1029-1037. Microsomes from gut epithelial cells can also be prepared using calcium precipitation as described in Bonkovsky, H. L. et al., Gastroenterology 1985; 88:458-467. Microsomes can be incubated with drugs and the metabolites monitored as a function of time. In addition the levels of these enzymes in tissue samples can be measured using radioimmunoassays or western blots.

Isolated microsomes can be used to determine inhibition of CYP3A drug biotransformation. Generally, the drug will be a substrate of CYP3A. The addition of the inhibitor will decrease the ability of CYP3A to catalyze drug metabolism. Inhibitors identified in this assay will be inhibitors of CYP3A function and diminish substrate catalysis. The production of metabolites can be monitored using high pressure liquid chromatography systems (HPLC) and identified based on retention times. CYP3A activity can also be assayed by calorimetrically measuring erythromycin demethylase activity as the production of formaldehyde as in Wrighton, et al., Mol. Pharmacol. 1985; 28:312-321 and Nash, T., Biochem. J. 1953; 55:416-421.

### Methods of Treatment

Methods of treating, preventing, or ameliorating disorders such as viral infections are provided herein. In practicing the methods, effective amounts of a prodrug, e.g. of an anti-viral compound, in particular, an antiviral lipid-containing compound and an enhancer, sequentially or in combination, are administered. The compounds may be administered in any desired manner, e.g., via oral, rectal, nasal, topical (including buccal and sublingual), vaginal, or parenteral (including subcutaneous, intramuscular, subcutaneous, intravenous, intradermal, intraocular, intratracheal, intracisternal, intraperitoneal, and epidural) administration. The compounds may be administered in combination or alternation by the same or different route of administration.

In certain embodiments, the viral infections that can be treated include influenza; pestiviruses such as bovine viral diarrhea virus (BVDV), classic swine fever virus (CSFV, also known as hog cholera virus), and Border disease virus of sheep (BDV); flaviviruses like dengue hemorrhagic fever virus (DHF or DENV), yellow fever virus (YFV), West Nile virus (WNV), shock syndrome and Japanese encephalitis virus; hepatitis B and C virus; cytomegalovirus (CMV); herpes infections, such as those caused by Varicella zoster virus, Herpes simplex virus types 1 & 2, human herpes virus 6, Epstein-Barr virus, Herpes type 6 (HHV-6) and type 8 (HHV-8); Varicella zoster virus infections such as shingles or chicken pox; Epstein Barr virus infections, including, but not limited to infectious mononucleosis/glandular; retroviral infections including, but not limited to SIV, HIV-1 and HIV-2; ebola virus; adenovirus and papilloma virus.

Specific flaviviruses further include, without limitation: Absettarov, Alfuy, Apoi, Aroa, Bagaza, Banzi, Bouboui, Bussuquara, Cacipacore, Carey Island, Dakar bat, Dengue 1, Dengue 2, Dengue 3, Dengue 4, Edge Hill, Entebbe bat, Gadgets Gully, Hanzalova, Hypr, Ilheus, Israel turkey meningoencephalitis, Japanese encephalitis, Jugra, Jutiapa, Kadam, Karshi, Kedougou, Kokobera, Koutango, Kumlinge, Kunjin, Kyasanur Forest disease, Langat, Louping ill, Meaban, Modoc, Montana myotis leukoencephalitis, Murray valley encephalitis, Naranjal, Negishi, Ntaya, Omsk hemorrhagic fever, Phnom-Penh bat, Powassan, Rio Bravo, Rocio, Royal Farm, Russian spring-summer encephalitis, Saboya, St. Louis encephalitis, Sal Vieja, San Perlita, Saumarez Reef, Sepik, Sokuluk, Spondweni, Stratford, Tembusu, Tyuleniy, Uganda S, Usutu, Wesselsbron, West Nile, Yaounde, Yellow fever, and Zika.

In further embodiments, the anti-viral compounds and the enhancer are administered in an effective amount for the treatment or prophylaxis of viral infections resulting from orthopox viruses, such as variola major and minor, vaccinia, molluscum contagiosum, orf (ecthyma contagiosum) smallpox, cowpox, camelpox, mousepox, rabbitpox, and monkeypox.

In one embodiment, a therapeutically effective dosage to treat such an orthopox infection should produce a serum concentration of anti-viral agent of about 0.1 ng/ml to about 50- 100 µg/ml. The pharmaceutical compositions, in another embodiment, should provide a dosage of from about 0.001 mg to about 2000 mg of compound per kilogram of body weight per day. Pharmaceutical dosage unit forms are prepared, e.g., to provide from about 0.01 mg, 0.1 mg or 1 mg to about 500mg, 1000 mg or 2000 mg, and in one embodiment from about 10 mg to about 500 mg of the active ingredient or a combination of essential ingredients per dosage unit form.

The amount of the enhancer can be selected using methods known in the art to enhance the bioavailability of the anti-viral agent. Any amount can be used that provides an desired response. The dosages may range, in a non-limiting example, from 0.001 mg to about 2000 mg of compound per kilogram of body weight per day, e.g. 0.01 to 500 mg/kg, or e.g, 0,1-10 mg/kg.

### Combination Therapy

The compounds and compositions provided herein may also be used in combination, and alternatively, in combination with other active ingredients. In certain embodiments, the compounds may be administered in combination, or sequentially, with another therapeutic agent. Such other therapeutic agents include those known for treatment, prevention, or amelioration of one or more symptoms associated with viral infections. It should be understood that any suitable combination of the compounds provided herein with one or more of the above-mentioned compounds and optionally one or more further pharmacologically active substances are considered to be within the scope of the present disclosure. In another embodiment, the compound provided herein is administered prior to or subsequent to the one or more additional active ingredients. In one embodiment, two or more of the antiviral agents disclosed herein are administered serially or in combination.

### Pharmaceutical Compositions

Pharmaceutical carriers suitable for administration of the compounds provided herein include any such carriers known to those skilled in the art to be suitable for the particular mode of administration. The compounds may be formulated as the sole pharmaceutically active ingredient in the composition or may be combined with other active ingredients.

Compositions comprising the compounds disclosed herein may be suitable for oral, rectal, nasal, topical (including buccal and sublingual), vaginal, or parenteral (including subcutaneous, intramuscular, subcutaneous, intravenous, intradermal, intraocular, intratracheal, intracisternal, intraperitoneal, and epidural) administration.

The compositions may conveniently be presented in unit dosage form and may be prepared by conventional pharmaceutical techniques. Such techniques include the step of bringing into association one or more compositions provided herein and one or more pharmaceutical carriers or excipients.

The compounds can be formulated into suitable pharmaceutical preparations such as solutions, suspensions, tablets, dispersible tablets, pills, capsules, powders, sustained release formulations or elixirs, for oral administration or in sterile solutions or suspensions for parenteral administration, as well as transdermal patch preparation and dry powder inhalers. In one embodiment, the compounds described above are formulated into pharmaceutical compositions using techniques and procedures well known in the art (see, e.g., Ansel Introduction to Pharmaceutical Dosage Forms, Fourth Edition 1985, 126).

In the compositions, effective concentrations of one or more compounds or pharmaceutically acceptable derivatives thereof may be mixed with one or more suitable pharmaceutical carriers. The compounds may be derivatized as the corresponding salts, esters, enol ethers or esters, acetals, ketals, orthoesters, hemiacetals, hemiketals, acids, bases, solvates, hydrates or prodrugs prior to formulation. The concentrations of the compounds in the compositions are effective for delivery of an amount, upon administration, that treats, prevents, or ameliorates one or more of the symptoms of the target disease or disorder. In one embodiment, the compositions are formulated for single dosage administration. To formulate a composition, the weight fraction of compound is dissolved, suspended, dispersed or otherwise mixed in a selected carrier at an effective concentration such that the treated condition is relieved, prevented, or one or more symptoms are ameliorated.

Compositions suitable for oral administration may be presented as discrete units such as, but not limited to, tablets, caplets, pills or dragees capsules, or cachets, each containing a predetermined amount of one or more of the compositions; as a powder or granules; as a solution or a suspension in an aqueous liquid or a non-aqueous liquid; or as an oil-in-water liquid emulsion or a water-in-oil emulsion or as a bolus, etc.

Liquid pharmaceutically administrable compositions can, for example, be prepared by dissolving, dispersing, or otherwise mixing an active compound as defined above and optional pharmaceutical adjuvants in a carrier, such as, for example, water, saline, aqueous dextrose, glycerol, glycols, ethanol, and the like, to thereby form a solution or suspension. If desired, the pharmaceutical composition to be administered may also contain minor amounts of nontoxic auxiliary substances such as wetting agents, emulsifying agents, solubilizing agents, pH buffering agents, preservatives, flavoring agents, and the like, for example, acetate, sodium citrate, cyclodextrine derivatives, sorbitan monolaurate, triethanolamine sodium acetate, triethanolamine oleate, and other such agents. Methods of preparing such dosage forms are known, or will be apparent, to those skilled in this art; for example, see Remington's Pharmaceutical Sciences, Mack Publishing Company, Easton, Pa., 15th Edition, 1975.

Compositions of the present invention suitable for topical administration in the mouth include for example, lozenges, having the ingredients in a flavored basis, usually sucrose and acacia or tragacanth; pastilles, having one or more of the compositions of the present invention in an inert basis such as gelatin and glycerin, or sucrose and acacia; and mouthwashes, having one or more of the compositions of the present invention administered in a suitable liquid carrier.

The tablets, pills, capsules, troches and the like can contain one or more of the following ingredients, or compounds of a similar nature: a binder; a lubricant; a diluent; a glidant; a disintegrating agent; a coloring agent; a sweetening agent; a flavoring agent; a wetting agent; an emetic coating; and a film coating. Examples of binders include microcrystalline cellulose, gum tragacanth, glucose solution, acacia mucilage, gelatin solution, molasses, polvinylpyrrolidine, povidone, crospovidones, sucrose and starch paste. Lubricants include talc, starch, magnesium or calcium stearate, lycopodium and stearic acid. Diluents include, for example, lactose, sucrose, starch, kaolin, salt, mannitol and dicalcium phosphate. Glidants include, but are not limited to, colloidal silicon dioxide. Disintegrating agents include crosscarmellose sodium, sodium starch glycolate, alginic acid, corn starch, potato starch, bentonite, methylcellulose, agar and carboxymethylcellulose. Coloring agents include, for example, any of the approved certified water soluble FD and C dyes, mixtures thereof; and water insoluble FD and C dyes suspended on alumina hydrate. Sweetening agents include sucrose, lactose, mannitol and artificial sweetening agents such as saccharin, and any number of spray dried flavors. Flavoring agents include natural flavors extracted from plants such as fruits and synthetic blends of compounds which produce a pleasant sensation, such as, but not limited to peppermint and methyl salicylate. Wetting agents include propylene glycol monostearate, sorbitan monooleate, diethylene glycol monolaurate and polyoxyethylene laural ether. Emetic-coatings include fatty acids, fats, waxes, shellac, ammoniated shellac and cellulose acetate phthalates. Film coatings include hydroxyethylcellulose, sodium carboxymethylcellulose, polyethylene glycol 4000 and cellulose acetate phthalate.

Compositions suitable for topical administration to the skin may be presented as ointments, creams, gels, and pastes, having one or more of the compositions administered in a pharmaceutical acceptable carrier.

Compositions for rectal administration may be presented as a suppository with a suitable base comprising, for example, cocoa butter or a salicylate.

Compositions suitable for nasal administration, when the carrier is a solid, include a coarse powder having a particle size, for example, in the range of 20 to 500 microns which is administered in the manner in which snuff is taken, (i.e., by rapid inhalation through the nasal passage from a container of the powder held close up to the nose). When the carrier is a liquid (for example, a nasal spray or as nasal drops), one or more of the compositions can be admixed in an aqueous or oily solution, and inhaled or sprayed into the nasal passage.

Compositions suitable for vaginal administration may be presented as pessaries, tampons, creams, gels, pastes, foams or spray formulations containing one or more of the compositions and appropriate carriers.

Compositions suitable for parenteral administration include aqueous and non-aqueous sterile injection solutions which may contain anti-oxidants, buffers, bacteriostats, and solutes which render the formulation isotonic with the blood of the intended recipient; and aqueous and non-aqueous sterile suspensions which may include suspending agents and thickening agents. The compositions may be presented in unit-dose or multi-dose containers, for example, sealed ampules and vials, and may be stored in a freeze-dried (lyophilized) condition requiring only the addition of the sterile liquid carrier, for example, water for injections, immediately prior to use. Extemporaneous injection solutions and suspensions may be prepared from sterile powders, granules, and tablets of the kind previously described above.

Pharmaceutical organic or inorganic solid or liquid carrier media suitable for enteral or parenteral administration can be used to fabricate the compositions. Gelatin, lactose, starch, magnesium stearate, talc, vegetable and animal fats and oils, gum, polyalkylene glycol, water, or other known carriers may all be suitable as carrier media.

Compositions may be used as the active ingredient in combination with one or more pharmaceutically acceptable carrier mediums and/or excipients. As used herein, "pharmaceutically acceptable carrier" includes any and all carriers, solvents, diluents, or other liquid vehicles, dispersion or suspension aids, surface active agents, isotonic agents, thickening or emulsifying agents, preservatives, solid binders, lubricants, adjuvants, vehicles, delivery systems, disintegrants, absorbents, preservatives, surfactants, colorants, flavorants, or sweeteners and the like, as suited to the particular dosage form desired.

Additionally, the compositions may be combined with pharmaceutically acceptable excipients, and, optionally, sustained-release matrices, such as biodegradable polymers, to form therapeutic compositions. A "pharmaceutically acceptable excipient" includes a non-toxic solid, semi-solid or liquid filler, diluent, encapsulating material or formulation auxiliary of any type.

It will be understood, however, that the total daily usage of the compositions will be decided by the attending physician within the scope of sound medical judgment. The specific therapeutically effective dose level for any particular host will depend upon a variety of factors, including for example, the disorder being treated and the severity of the disorder; activity of the specific composition employed; the specific composition employed, the age, body weight, general health, sex and diet of the patient; the time of administration; route of administration; rate of excretion of the specific compound employed; the duration of the treatment; drugs used in combination or coincidential with the specific composition employed; and like factors well known in the medical arts. For example, it is well within the skill of the art to start doses of the composition at levels lower than those required to achieve the desired therapeutic effect and to gradually increase the dosage until the desired effect is achieved.

Compositions are preferably formulated in dosage unit form for ease of administration and uniformity of dosage. "Dosage unit form" as used herein refers to a physically discrete unit of the composition appropriate for the host to be treated. Each dosage should contain the quantity of composition calculated to produce the desired therapeutic affect either as such, or in association with the selected pharmaceutical carrier medium.

Exemplary unit dosage formulations are those containing a daily dose or unit, daily sub-dose, or an appropriate fraction thereof, of the administered ingredient. The dosage will depend on host factors such as weight, age, surface area, metabolism, tissue distribution, absorption rate and excretion rate. Exemplary systemic dosages for all of the herein described conditions are those ranging from 0.01 mg/kg to 2000 mg/kg of body weight per day as a single daily dose or divided daily doses. Typical dosages for topical application are those ranging from 0.001 to 100% by weight of the active compound.

The therapeutically effective dose level will depend on many factors as noted above. In addition, it is well within the skill of the art to start doses of the composition at relatively low levels, and increase the dosage until the desired effect is achieved.

Compositions comprising a compound disclosed herein may be used with a sustained-release matrix, which can be made of materials, usually polymers, which are degradable by enzymatic or acid-based hydrolysis or by dissolution. Once inserted into the body, the matrix is acted upon by enzymes and body fluids. A sustained-release matrix for example is chosen from biocompatible materials such as liposomes, polylactides (polylactic acid), polyglycolide (polymer of glycolic acid), polylactide co-glycolide (copolymers of lactic acid and glycolic acid), polyanhydrides, poly(ortho)esters, polypeptides, hyaluronic acid, collagen, chondroitin sulfate, carboxcylic acids, fatty acids, phospholipids, polysaccharides, nucleic acids, polyamino acids, amino acids such as phenylalanine, tyrosine, isoleucine, polynucleotides, polyvinyl propylene, polyvinylpyrrolidone and silicone. A preferred biodegradable matrix is a matrix of one of either polylactide, polyglycolide, or polylactide co-glycolide (copolymers of lactic acid and glycolic acid).

The compounds may also be administered in the form of liposomes. As is known in the art, liposomes are generally derived from phospholipids or other lipid substances. Liposomes are formed by mono- or multi-lamellar hydrated liquid crystals that are dispersed in an aqueous medium. Any non-toxic, physiologically-acceptable and metabolizable lipid capable of forming liposomes can be used. The liposome can contain, in addition to one or more compositions of the present invention, stabilizers, preservatives, excipients, and the like. Examples of lipids are the phospholipids and the phosphatidyl cholines (lecithins), both natural and synthetic. Methods to form liposomes are known in the art.

The compounds may be formulated as aerosols for application, such as by inhalation. These formulations for administration to the respiratory tract can be in the form of an aerosol or solution for a nebulizer, or as a microfine powder for insufflation, alone or in combination with an inert carrier such as lactose. In such a case, the particles of the formulation will, in one embodiment, have diameters of less than 50 microns, in one embodiment less than 10 microns.

Compositions comprising the compounds disclosed herein may be used in combination with other compositions and/or procedures for the treatment of the conditions described above.

### Definitions

The term "alkyl", as used herein, unless otherwise specified, includes a saturated straight, branched, or cyclic, primary, secondary, or tertiary hydrocarbon, of, e.g., C₁₋₃₀ or C₁₋₂₂, and specifically includes methyl, ethyl, propyl, isopropyl, cyclopropyl, butyl, isobutyl, secbutyl, *t*-butyl, pentyl, cyclopentyl, isopentyl, neopentyl, hexyl, isohexyl, cyclohexyl, cyclohexylmethyl, heptyl, cycloheptyl, octyl, cyclo-octyl, dodecyl, tridecyl, pentadecyl, icosyl, hemicosyl, and decosyl. The alkyl group may be optionally substituted with, e.g., halogen (fluoro, chloro, bromo or iodo), hydroxyl, amino, alkylamino, arylamino, alkoxy, aryloxy, nitro, cyano, sulfonic acid, sulfate, phosphonic acid, phosphate, or phosphonate, either unprotected, or protected as necessary, as known to those skilled in the art, for example, as taught in Greene, et al., Protective Groups in Organic Synthesis, John Wiley and Sons, Second Edition, 1991, hereby incorporated by reference.

The term "lower alkyl", as used herein, and unless otherwise specified, includes a C₁ to C₄ saturated straight, branched, or if appropriate, a cyclic (for example, cyclopropyl) alkyl group, which is optionally substituted.

Whenever a range of carbon atoms is referred to, it includes independently and separately every member of the range. As a nonlimiting example, the term "C₁-C₁₀ alkyl" is considered to include, independently, each member of the group, such that, for example, C₁-C₁₀ alkyl includes straight, branched and where appropriate cyclic C₁, C₂, C₃, C₄, C₅, C₆, C₇, C₈, C₉ and C₁₀ alkyl functionalities.

The term "protected" as used herein and unless otherwise defined includes a group that is added to an atom such as an oxygen, nitrogen, or phosphorus atom to prevent its further reaction or for other purposes. A wide variety of oxygen and nitrogen protecting groups are known to those skilled in the art of organic synthesis.

The term "halo", as used herein, specifically includes to chloro, bromo, iodo, and fluoro.

The term "alkenyl" includes a straight, branched, or cyclic hydrocarbon of, for example, C₂₋₁₀₀, or C₂₋₂₂ with at least one double bond. Examples include, but are not limited to, vinyl, allyl, and methyl-vinyl. The alkenyl group can be optionally substituted in the same manner as described above for the alkyl groups.

The term "alkynyl" includes, for example, a C₂₋₁₀₀ or C₂₋₂₂ straight or branched hydrocarbon with at least one triple bond. The alkynyl group can be optionally substituted in the same manner as described above for the alkyl groups.

The term "alkoxy" includes a moiety of the structure -O-alkyl.

The term "acyl" includes a group of the formula R'C(O), wherein R' is a straight, branched, or cyclic, substituted or unsubstituted alkyl or aryl.

As used herein, "aryl" includes aromatic groups having in the range of 6 up to 14 carbon atoms and "substituted aryl" refers to aryl groups further bearing one or more substituents as set forth above.

As used herein, "heteroaryl" includes aromatic groups containing one or more heteroatoms (e.g., N, O, S, or the like) as part of the ring structure, and having in the range of 3 up to 14 carbon atoms and "substituted heteroaryl" refers to heteroaryl groups further bearing one or more substituents as set forth above.

As used herein, the term "bond" or "valence bond" includes a linkage between atoms consisting of an electron pair.

The term "host", as used herein, unless otherwise specified, includes mammals (e.g., cats, dogs, horses, mice, monkeys, etc.), humans, or other organisms in need of treatment. The host is for example, a human or an animal, including without limitation, primates, including macaques, baboons, as wells as chimpanzee, gorilla, and orangutan, ruminants, including sheep, goats, deer, and cattle, for example, cows, steers, bulls, and oxen; swine, including pigs; and poultry including chickens, turkeys, ducks, or geese.

The term "pharmaceutically acceptable salt" as used herein, unless otherwise specified, includes those salts which are, within the scope of sound medical judgment, suitable for use in contact with the tissues of hosts without undue toxicity, irritation, allergic response and the like, and are commensurate with a reasonable benefit/risk ratio and effective for their intended use. The salts can be prepared in situ during the final isolation and purification of one or more compounds of the composition, or separately by reacting the free base function with a suitable organic acid. Non-pharmaceutically acceptable acids and bases also find use herein, as for example, in the synthesis and/or purification of the compounds of interest. Nonlimiting examples of such salts are (a) acid addition salts formed with inorganic salts (for example hydrochloric acid, hydrobromic acid, sulfuric acid, phosphoric acid, nitric acid, and the like), and salts formed with organic salts such as acetic acid, oxalic acid, tartaric acid, succinic acid, ascorbic acid, benzoic acid, tannic acid, and the like; (b) base addition salts formed with metal cations such as zinc, calcium, magnesium, aluminum, sodium, potassium, copper, nickel and the like; (c) combinations of (a) and (b). Also included as "pharmaceutically acceptable salts" are amine salts.

The term "pharmaceutically acceptable esters" as used herein, unless otherwise specified, includes those esters of one or more compounds, which are, within the scope of sound medical judgment, suitable for use in contact with the tissues of hosts without undue toxicity, irritation, allergic response and the like, are commensurate with a reasonable benefit/risk ratio, and are effective for their intended use.

The term "pharmaceutically acceptable prodrug" includes a compound that is metabolized, for example, hydrolyzed or oxidized, in the host to form an active compound. Typical examples of prodrugs include compounds that have biologically labile protecting groups on a functional moiety of the active compound. Prodrugs include compounds that can be oxidized, reduced, aminated, deaminated, hydroxylated, dehydroxylated, hydrolyzed, dehydrolyzed, alkylated, dealkylated, acylated, deacylated, phosphorylated, dephosphorylated to produce the active compound.

The term "enantiomerically enriched", as used herein, refers to a compound that is a mixture of enantiomers in which one enantiomer is present in excess, and preferably present to the extent of 95% or more, and more preferably 98% or more, including 100%.

The term "effective amount" includes an amount required for prevention, treatment, or amelioration of one or more of the symptoms of diseases or disorders provided herein.

It is to be understood that the compounds disclosed herein may contain chiral centers. Such chiral centers may be of either the (R) or (S) configuration, or may be a mixture thereof. Thus, the compounds provided herein may be enantiomerically pure, or be stereoisomeric or diastereomeric mixtures. It is understood that the disclosure of a compound herein encompasses any racemic, optically active, polymorphic, or steroisomeric form, or mixtures thereof, which preferably possesses the useful properties described herein, it being well known in the art how to prepare optically active forms and how to determine activity using the standard tests described herein, or using other similar tests which are will known in the art. Examples of methods that can be used to obtain optical isomers of the compounds include the following:
i) physical separation of crystals- a technique whereby macroscopic crystals of the individual enantiomers are manually separated. This technique can be used if crystals of the separate enantiomers exist, i.e., the material is a conglomerate, and the crystals are visually distinct;
ii) simultaneous crystallization- a technique whereby the individual enantiomers are separately crystallized from a solution of the racemate, possible only if the latter is a conglomerate in the solid state;
iii) enzymatic resolutions, a technique whereby partial or complete separation of a racemate by virtue of differing rates of reaction for the enantiomers with an enzyme;
iv) enzymatic asymmetric synthesis, a synthetic technique whereby at least one step of the synthesis uses an enzymatic reaction to obtain an enantiomerically pure or enriched synthetic precursor of the desired enantiomer;
v) chemical asymmetric synthesis, a synthetic technique whereby the desired enantiomer is synthesized from an achiral precursor under conditions that produce assymetry (i.e., chirality) in the product, which may be achieved using chiral catalysts or chiral auxiliaries;
vi) diastereomer separations-a technique whereby a racemic compound is reacted with an enantiomerically pure reagent (the chiral auxiliary) that converts the individual enantiomers to diastereomers. The resulting diastereomers are then separated by chromatography or crystallization by virtue of their now more distinct structural differences and the chiral auxiliary later removed to obtain the desired enantiomer;
vii) first- and second-order asymmetric transformations, a technique whereby diastereomers from the racemate equilibrate to yield a preponderance in solution of the diastereomer from the desired enantiomer or where preferential crystallization of the diastereomer from the desired enantiomer perturbs the equilibrium such that eventually in principle all the material is converted to the crystalline diastereomer from the desired enantiomer. The desired enantiomer is then released from the diastereomer;
viii) kinetic resolutions - this technique refers to the achievement of partial or complete resolution of a racemate (or of a further resolution of a partially resolved compound) by virtue of unequal reaction rates of the enantiomers with a chiral, non-racemic reagent or catalyst under kinetic conditions;
ix) enantiospecific synthesis from non-racemic precursors-a synthetic technique whereby the desired enantiomer is obtained from non-chiral starting materials and where the stereochemical integrity is not or is only minimally compromised over the course of the synthesis;
x) chiral liquid chromatography-a technique whereby the enantiomers of a racemate are separated in a liquid mobile phase by virtue of their differing interactions with a stationary phase. The stationary phase can be made of chiral material or the mobile phase can contain an additional chiral material to provoke the differing interactions;
xi) chiral gas chromatography-a technique whereby the racemate is volatilized and enantiomers are separated by virtue of their differing interactions in the gaseous mobile phase with a column containing a fixed non-racemic chiral adsorbent phase;
xii) extraction with chiral solvents-a technique whereby the enantiomers are separated by virtue of preferential dissolution of one enantiomer into a particular chiral solvent;
xiii) transport across chiral membranes-a technique whereby a racemate is placed in contact with a thin membrane barrier. The barrier typically separates two miscible fluids, one containing the racemate, and a driving force such as concentration or pressure differential causes preferential transport across the membrane barrier. Separation occurs as a result of the non-racemic chiral nature of the membrane which allows only one enantiomer of the racemate to pass through.

### Synthesis of Antiviral Compounds

Antiviral compounds, and in particular antiviral lipid-containing compounds may be synthesized using methods available in the art. As described in U.S. Patent No. 6,716,825, the disclosure of which is incorporated herein by reference. The antiviral compounds and lipid containing prodrugs provided herein can be prepared in a variety of ways, as generally depicted in Schemes I-II. The general phosphonate esterification methods described below are provided for illustrative purposes only and are not to be construed as limiting in any manner. Indeed, several methods have been developed for direct condensation of phosphonic acids with alcohols (see, for example, R. C. Larock, Comprehensive Organic Transformations, VCH, New York, 1989, p. 966 and references cited therein). Isolation and purification of the compounds and intermediates described in the examples can be effected, if desired, by any suitable separation or purification procedure such as, for example, filtration, extraction, crystallization, flash column chromatography, thin-layer chromatography, distillation or a combination of these procedures. Specific illustrations of suitable separation and isolation procedures are in the examples below. Other equivalent separation and isolation procedures can of course, also be used.

Scheme I illustrates a general synthesis of alkylglycerol or alkylpropanediol analogs of cidofovir, cyclic cidofovir, and other phosphonates. Treatment of 2,3-isopropylidene glycerol, 1, with NaH in dimethylformamide followed by reaction with an alkyl methanesulfonate yields the alkyl ether, 2. Removal of the isopropylidene group by treatment with acetic acid followed by reaction with trityl chloride in pyridine yields the intermediate 3. Alkylation of intermediate 3 with an alkyl halide results in compound 4. Removal of the trityl group with 80% aqueous acetic acid affords the O,O-dialkyl glycerol, 5. Bromination of compound 5 followed by reaction with the sodium salt of cyclic cidofovir or other phosphonate-containing nucleotide yields the desired phosphonate adduct, 7. Ring-opening of the cyclic adduct is accomplished by reaction with aqueous sodium hydroxide. The preferred propanediol species may be synthesized by substituting 1-O-alkylpropane-3-ol for compound 5 in Scheme I. The tenofovir and adefovir analogs may be synthesized by substituting these nucleotide phosphonates for cCDV in reaction (f) of Scheme I. Similarly, other nucleotide phosphonates may be formed in this manner.

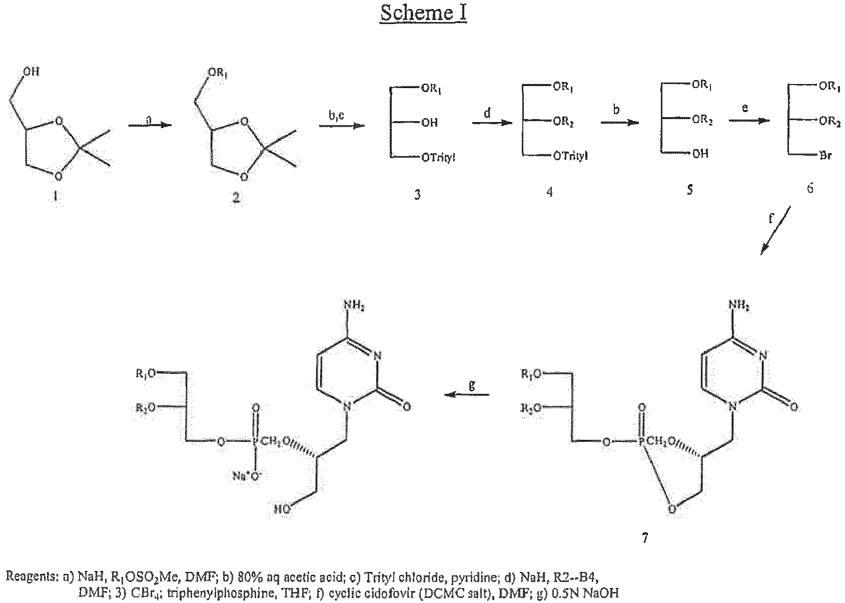

Scheme II illustrates a general method for the synthesis of nucleotide phosphonates using 1-O-hexadecyloxypropyl-adefovir as the example. The nucleotide phosphonate (5 mmol) is suspended in dry pyridine and an alkoxyalkanol or alkylglycerol derivative (6 mmol) and 1,3-dicyclohexylcarbodiimde (DCC, 10 mmol) are added. The mixture is heated to reflux and stirred vigorously until the condensation reaction is complete as monitored by thin-layer chromatography. The mixture is then cooled and filtered. The filtrate is concentrated under reduced pressure and the residue s adsorbed on silica gel and purified by flash column chromatography (elution with approx. 9:1 dichloromethane/methanol) to yield the corresponding phosphonate monoester.

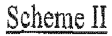

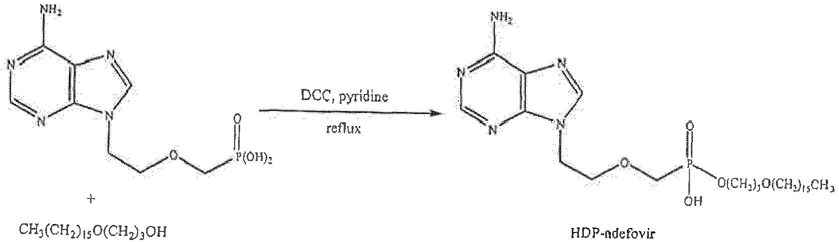

The invention will be further understood from the following non-limiting examples.

### EXAMPLES

### Example 1

(As described in U.S. Patent No. 6,716,825)

### Synthesis of Adefovir Hexadecyloxypropyl and 1-O-Octadecyl-sn-glyceryl Esters

To a mixture of adefovir (1.36 g, 5 mmol) and 3-hexadecyloxy-1-propanol (1.8 g, 6 mmol) in dry pyridine was added DCC (2.06 g, 10 mmol). The mixture was heated to reflux and stirred 18 h then cooled and filtered. The filtrate was concentrated under reduced pressure and the residue was applied to a short column of silica gel. Elution of the column with 9:1 dichloromethane/methanol yielded hexadecyloxypropyl-adefovir (HDP-ADV) as a white powder.

To a mixture of adefovir (1.36 g, 5 mmol) and 1-O-octadecyl-sn-glycerol (2.08 g, 6 mmol) in dry pyridine (30 mL) was added DCC (2.06 g, 10 mmol). The mixture was heated to reflux and stirred overnight then cooled and filtered. The filtrate was concentrated under reduced pressure and the residue was applied to a column of silica gel. Elution of the column with a 9:1 dichloromethane/methanol yielded 1-O-octadecyl-sn-glyceryl-3-adefovir.

### Example 2

(As described in U.S. Patent No. 6,716,825)

### Synthesis of AZT-phosphonate Hexadecyloxypropyl Ester

The phosphonate analog of AZT (3'-Azido-3'-5'-dideoxythymidine-5'-phosphonic acid) was synthesized using the published procedure: Hakimelahi, G. H.; Moosavi-Movahedi, A. A.; Sadeghi, M. M.; Tsay, S-C.; Hwu, J. R. Journal of Medicinal Chemistry, 1995 38, 4648-4659.

The AZT phosphonate (1.65 g, 5 mmol) was suspended in dry pyridine (30 mL), then 3-hexadecyloxy-1-propanol (1.8 g, 6 mmol) and DCC (2.06 g, 10 mmol) were added and the mixture was heated to reflux and stirred for 6 h, then cooled and filtered. The filtrate was concentrated under reduced pressure and the residue was applied to a column of silica gel. Elution of the column with a 9:1 dichloromethane/methanol yielded 3'-azido-3'-5'-dideoxythymidine-5'-phosphonic acid, hexadecyloxypropyl ester.

### Example 3

(As described in U.S. Patent No. 6,716,825)

### Synthesis of the Hexadecyloxypropyl, Octadecyloxypropyl, Octadecyloxyethyl and Hexadecyl Esters of Cyclic Cidofovir

To a stirred suspension of cidofovir (1.0 g, 3.17 mmol) in N,N-DMF (25 mL) was added N,N-dicyclohexyl-4-morpholine carboxamidine (DCMC, 1.0 g, 3.5 mmol). The mixture was stirred overnight to dissolve the cidofovir. This clear solution was then charged to an addition funnel and slowly added (30 min.) to a stirred, hot pyridine solution (25 mL, 60°C.) of 1,3-dicyclohexyl carbodiimide (1.64 g, 7.9 mmol). This reaction mixture was stirred at 100°C for 16 h then cooled to room temperature, and the solvent was removed under reduced pressure. The residue was adsorbed on silica gel and purified by flash column chromatography using gradient elution (CH₂Cl₂ +MeOH). The UV active product was finally eluted with 5:5:1 CH₂Cl₂ /MeOH/H₂O Evaporation of the solvent gave 860 mg of a white solid. The ¹H and ³¹P NMR spectrum showed this to be the DCMC salt of cyclic cidofovir (yield=44%).

To a solution of cyclic cidofovir (DCMC salt) (0.5 g, 0.8 mmol) in dry DMF (35 mL) was added 1-bromo-3-hexadecyloxypropane (1.45 g, 4 mmol) and the mixture was stirred and heated at 80°C for 6 h. The solution was then concentrated in vacuo and the residue adsorbed on silica gel and purified by flash column chromatography using gradient elution (CH₂Cl₂ +EtOH). The alkylated product was eluted with 90:10 CH₂Cl₂ /EtOH. The fractions containing pure product were evaporated to yield 260 mg HDP-cyclic cidofovir (55% yield).

To a solution of cyclic cidofovir (DCMC salt) (1.0 g, 3.7 mmol) in dry DMF (35 mL) was added 1-bromo-3-octadecyloxypropane (2.82 g, 7.2 mmol) and the mixture was stirred and heated at 85°C for 5h. The solution was then concentrated in vacuo and the residue adsorbed on silica gel and purified by flash column chromatography using gradient elution (CH₂Cl₂ +MeOH). The alkylated product was eluted with 9:1 CH₂Cl₂ /MeOH. The fractions containing pure product were evaporated to yield 450 mg ODP-cyclic cidofovir.

To a solution of cCDV (DCMC salt) (1.0 g, 3.7 mmol) in dry DMF (35 mL) was added 1-bromo-3-octadecyloxyethane (3.0 g, 7.9 mmol) and the mixture was stirred and heated at 80°C for 4 h. The solution was then concentrated in vacuo and the residue adsorbed on silica gel and purified by flash column chromatography using gradient elution (CH₂Cl₂ +MeOH). The alkylated product was eluted with 9:1 CH₂Cl₂ /MeOH. The fractions containing pure product were evaporated to yield 320 mg octadecyloxyethyl-cCDV.

To a solution of cyclic cidofovir (DCMC salt) (0.5 g, 0.8 mmol) in dry DMF (35 mL) was added 1-bromo-hexadecane (1.2 g, 4 mmol) and the mixture was stirred and heated at 80°C for 6 h. The solution was then concentrated in vacuo and the residue adsorbed on silica gel and purified by flash column chromatography using gradient elution (CH₂Cl₂ +MeOH). The alkylated product was eluted with 9:1 CH₂Cl₂ /MeOH. The fractions containing pure product were evaporated to yield 160 mg hexadecyl-cCDV.

### Example 4

(As described in U.S. Patent No. 6,716,825)

### Synthesis of the Hexadecyloxypropyl, Octadecyloxypropyl, Octadecyloxyethyl and Hexadecyl Esters of Cidofovir

Hexadecyloxypropyl-cyclic CDV from above was dissolved in 0.5M NaOH and stirred at room temp for 1.5 h. 50% aqueous acetic was then added dropwise to adjust the pH to about 9. The precipitated HDP-CDV was isolated by filtration, rinsed with water and dried, then recrystallized (3:1 p-dioxane/water) to give HDP-CDV. Similarly, the octadecyloxypropyl-, octadecyloxyethyl- and hexadecyl-cCDV esters were hydrolyzed using 0.5M NaOH and purified to give the corresponding cidofovir diesters.

### Example 5

(As described in U.S. Patent No. 6,716,825)

### Synthesis of cyclic-ganciclovir phosphonate Hexadecyloxypropyl Ester

The cyclic phosphonate analog of ganciclovir was prepared using the published procedure: (Reist, E. J.; Sturm, P. A.; Pong, R. Y.; Tanga, M. J. and Sidwell, R. W. Synthesis of acyclonucleoside phosphonates for evaluation as antiviral agents, p. 17-34. In J, C. Martin (ed.), Nucleotide Analogues as Antiviral Agents, American Chemical Society, Washington, D.C.). After conversion to the DCMC salt in DMF the cGCV phosphonate was treated with 1-bromo-3-hexadecyloxypropane and the mixture was heated to 80°C for 6 hours. Isolation of the alkylated product by flash chromatography yielded HDP-cyclic-GCV phosphonate.

### Example 6

(As described in U.S. Patent No. 6,716,825)

### Synthesis of ganciclovir phosphonate hexadecyloxypropyl ester

HDP-cyclic GCV phosphonate from above was dissolved in 0.5M NaOH and stirred at room temperature to convert it to the acyclic diester. The solution was neutralized with 50% aq acetic acid to precipitate the product which was recrystallized in 3:1 p-dioxane/water.

### Example 7

### Antiviral Activity and Selectivity of Phospbonate Nucleotide Analogs Against Human Cytomegalovirus (HCMV)

HCMV antiviral assay: Antiviral assays for HCMV DNA were carried out by DNA hybridization as reported by Dankner, W. M., Scholl, D., Stanat, S. C., Martin, M., Souke, R. L. and Spector, S. A, J. Virol. Methods 21:293-298, 1990. Briefly, subconfluent MRC-5 cells in 24-well culture dishes were pretreated for 24 h with various concentrations of drug in Eagle s minimum essential medium (E-MEM) containing 2% FBS and antibiotics. The medium was removed and HCMV strains added at a dilution that will result in a 3-4+cytopathic effect (CPE) in the no-drug wells in 5 days. The virus was absorbed for 1' h at 37°C, aspirated and replaced with the drug dilutions. After 5 days of incubation HCMV DNA was quantified in triplicate by nucleic acid hybridization using a CMV Antiviral Susceptibility Test Kit from Diagnostic Hybrids, Inc. (Athens, Ohio). The medium was removed and cells lysed according to the manufacturer s instructions. After absorption of the lysate, the Hybriwix™ filters were hybridized overnight at 60°C. The Hybriwix™ were washed for 30 min at 73°C and counted in a gamma counter. The results are expressed as EC₅₀ (the 50% inhibitory concentration). Preliminary experiments were performed on 1-O-hexadecylpropanediol (HDP) derivatives of cidofovir and adefovir, as shown in Table 1.

**TABLE 1**

| Drug | HCMV | CEM | Selective Index |
|---|---|---|---|
| | EC_{50.}*µ*M | CC_{50.}*µ*M | |
| CDV | 0.45±0.09 (3) | 857 | 1,900 |
| cCDV | 0.47±0.13 (3) | >1000 | >2,100 |
| HDP-cCDV | 0.0005 (2) | 30 | 59,600 |
| Adefovir | 55 (1) | - | - |
| HDP-Adefovir | 0.01 (1) | - | - |

As the results in Table 1 indicate, 1-O-hexadecylpropanediol-3-cyclic CDV (HDP-cCDV) was >900 times more active than CDV or cyclic CDV. While more cytotoxic, the selectivity index against HCMV in rapidly dividing cells was >59,000 vs. 1,900 to >2,100 for the underivatized CDV's.

Cytotoxicity of test compounds in vitro: Subconfluent human lung fibroblast cells (MRC-5, American Type Culture Collection, Rockville, Md.) in 24-well plates were treated with drugs diluted in E-MEM (Gibco BRL, Grand Island, N.Y.) supplemented with 2% fetal bovine serum and antibiotics. After 5 days of incubation at 37°C, the cell monolayer was visually inspected under magnification and the concentration of drug which caused a 50% reduction in cell number was estimated.

The data obtained from these experiments is shown in Table 2.

**TABLE 2**

| Inhibition of Human CMV Replication in MRC-5 Human | | | |
|---|---|---|---|
| Lung Fibroblasts Assayed by DNA Reduction | | | |
| Compound | EC₅₀*.µ*M | CC_{50.,}*µ*M | Selective Index |
| 1 Cidofovir (CDV) | 0.46 | >1000 | >2174 |
| Cyclic Cidofovir (cCDV) | 0.47 | >1000 | >2128 |
| 1-O-hexadecylpropanediol-3-CDV | 2x10⁻⁶ | 10 | 5x10⁻⁶ |
| 1-O-hexadecylpropanediol-3-cCDV | 3x10⁻⁴ | 320 | 1x10⁻⁶ |
| 1-O-octadecylpropanediol-3-CDV | 3x10⁻⁵ | 32 | 1x10⁻⁶ |
| 1-O-octadecylpropanediol-3-cCDV | 3x10⁻⁴ | 320 | 1x10⁻⁶ |
| 1-O-octadecylpropanediol-2-CDV | 0.04 | 210 | 2x10⁻¹¹ |
| 1-O-octadecylpropanediol-2-cCDV | 55 | 320 | 1x10⁻⁶ |
| Hexadecyl-cCDV | 0.10 | 6.5 | 163 |
| Adefovir (ADV) | 0.21 | >1000 | >18 |
| 1-O-hexadecylpropanediol-3-ADV | | 6.5 | 65 |
| 1-O-glycero-3-ADV | | -- | -- |

| | | | |
|---|---|---|---|
| EC₅₀ - 50% effective concentration; CC₅₀ *-* 50% cytotoxic concentration; selectivity index - CC₅₀ /EC₅₀. EC₅₀ results are the average of 3 to 6 determinations, with the exception that ADV is a single replication done in duplicate | | | |

### Example 8

(As described in U.S. Patent No. 6,716,825)

### Effect of HDP-cCDV on Poxvirus Replication, in vitro

The activity of cidofovir (CDV), cyclic cidofovir (cCDV), and 1-O-hexadecylpropanediol-3-cCDV (HDP-cCDV) was tested for antiviral activity in human foreskin fibroblasts infected with vaccinia virus or cowpox virus by measuring the dose dependent reduction in cytopathic effect (CPE). Preliminary vaccinia and cowpox EC₅₀ values were determined in a CPE reduction assay in human foreskin fibroblast (HFF) cells. The data thus obtained is shown in Table 3.

| **TABLE 3** | | | |
|---|---|---|---|
| Drug | Vaccinia EC_{50, µM} | Cowpox, EC_{50, µM} | HFF Cells, CC_{50, µM} |
| CDV | 1.80 | 2.10 | 89.8 |
| Cyclic CDV | 0.97 | 0.72 | >100 |
| HDP-cCDV | 0.11 | <0.03 | >100 |
| Control lipid | >100 | >100 | >100 |

As shown in Table 3, HDP-cCDV was highly active against vaccinia virus with an IC₅₀ value of 0.11 µM versus 0.97 and 1.8 µM for cCDV and CDV, respectively. In cowpox infected cells HDP-cCDV was extremely effective with an IC₅₀ of <0.03 µM versus 0.72 and 2.1 for cCDV and CDV, respectively.
Poxvirus Antiviral Cytopathic Effect (CPE) Assay: At each drug concentration, three wells containing Vero cells were infected with 1000 pfu/well of orthopoxvirus and three others remained uninfected for toxicity determination. Plates were examined and stained after the virus-infected, untreated cells showed 4+CPE. Neutral red was added to the medium and CPE was assessed by neutral red uptake at 540 nm. The 50% inhibitory (EC₅₀) and cytotoxic concentrations (CC₅₀) were determined from plots of the dose response. The results are shown in Table 4.

| **TABLE 4** | | | | | | |
|---|---|---|---|---|---|---|
| EC_{50.}µM | | | | | | |
| Com-pound | Vac-cinia | Cow-pox | Variola Major, Bangladesh | Variola Major, Yamada | Variola Minor Garcia | CC₅₀ µM |
| CDV | 2.2 | 3.8 | 100 | _ | _ | >100 |
| cCDV | _ | _ | 100 | _ | _ | >100 |
| HDP-CDV | <0.03 | <0.03 | 0.0015 | 0.0015 | 0.0006 | >0.1 |
| HDP-cCDV | 0.11 | <0.03 | >0.01 | _ | _ | >0.1 |

| | | | | | | |
|---|---|---|---|---|---|---|
| EC₅₀ - 50% effective concentration, CC₅₀ - 50% cytotoxic concentration in Verocells; selectivity index - CC₅₀ /EC₅₀ ; Results are the average of 3 determinations. | | | | | | |

### Example 9

(As described in U.S. Patent No. 6,716,825)

### Effect of 1-O-Hexadecylpropanediol-3-Adefovir (HDP-ADV) on HIV-1 Replication, in vivo

Preliminary experiments in the inhibition of HIV-1 replication by compounds provided herein were performed as follows. Drug assays were carried out as previously described by Larder et. al., Antimicrobial Agents & Chemotherapy, 34:436-441, 1990. HIV-1_{LA1}, infected HT4-6C cells were exposed to drugs as indicated and incubated for 3 days at 37°C. The cells were fixed with crystal violet to visualize plaques. Antiviral activity was assessed as the percentage of control plaques (no drug) measured in drug treated samples. The EC₅₀ is the micromolar concentration which reduces plaque number by 50%. The activity of adefovir was compared with AZT (zidovudine) and 1-O-hexadecylpropanediol-3-adefovir (HDP-ADV) in HIV-1 infected HT4-6C cells. The results are shown in Table 5.

| **TABLE 5** | |
|---|---|
| Drug | EC_{50.} µM, in HIV-1 plaque reduction assay |
| AZT | 0.007 |
| Adefovir | 16.0 |
| HDP-ADV | 0.0001 |

Adefovir was moderately active with an EC₅₀ of 16 µM. AZT was highly active as anticipated (EC₅₀ 0.007 µM) but HDP-ADV was the most active of the three compounds with an EC₅₀ of 0.0001 µM, more than 5 logs more active than adefovir itself.

HIV-1 antiviral assay: The effect of antiviral compounds on HIV replication in CD4-expressing HeLa HT4-6C cells, was measured by a plaque reduction assay (Larder, B. A., Chesebro, B. and Richman, D. D. Antimirob. Agents Chemother., 34:436-441, 1990). Briefly, monolayers of HT4-6C cells were infected with 100-300 plaque forming units (PFU) of virus per well in 24-well microdilution plates. Various concentrations of drug were added to the culture medium, Dulbecco's modified Eagle medium containing 5% FBS and antibiotics, as noted above. After 3 days at 37°C, the monolayers were fixed with 10% formaldehyde solution in phosphate-buffered saline (PBS) and stained with 0.25% crystal violet to visualize virus plaques. Antiviral activity was assessed as the percentage of control plaques measured in drug-treated samples. Cytotoxicity was assessed by the method of Hostetler et al., Antiviral Research, 31:59-67, 1996. The results are shown in Table 6.

| **TABLE 6** | | | |
|---|---|---|---|
| Inhibition of HIV Replication in HT4-6C Cells by Plaque Reduction | | | |
| Compound | EC_{50,} µM | CC_{50,}µM | Selectivity index |
| Adefovir (ADV) | 8.2 | >1000 | >122 |
| 1-O-hexadecylpropanediol-3-ADV | 0.008 | 6.5 | 813 |

| | | | |
|---|---|---|---|
| EC₅₀ - 50% effective concentration; CC₅₀ - 50% cytotoxic concentration; selectivity index - CC₅₀ /EC₅₀ EC₅₀ values are the average of 4 experiments. | | | |

### Example 10

(As described in U.S. Patent No. 6,716,825)

### Effect of Cidofovir Analogs on Herpes Virus Replication

HSV-1 antiviral assay: Subconfluent MRC-5 cells in 24-well culture dishes were inoculated by removing the medium and adding HSV-1 virus at a dilution that will result in a 3-4+CPE in the no-drug well in 20-24 h. This was absorbed for 1 h at 37°C, aspirated and replaced with various concentrations of drugs in E-MEM containing 2% FBS and antibiotics. After approximately 24 h of incubation, HSV DNA was quantified in triplicate by nucleic acid hybridization using a HSV Antiviral Susceptibility Test Kit from Diagnostic Hybrids, Inc. (Athens, Ohio). The medium was removed and cells lysed according to the manufacturer s instructions. After absorption of the lysate, the Hybrwix™ filters were hybridized overnight at 60°C. The Hybriwix were washed for 30 min at 73°C and counted in a gamma counter. Cytotoxicity was assessed as described in Example 17. EC₅₀ and CC₅₀ values thus obtained are shown in Table 7.

| **TABLE 7** | | | |
|---|---|---|---|
| Inhibition of Human HSV Replication in MRC-5 | | | |
| Human Lung Fibroblasts Assayed by DNA Reduction | | | |
| Compound | EC_{50,} µM | CC_{50,} µM | Selectivity Index |
| Cidofovir (CDV) | 1.20 | >1000 | >800 |
| Cyclic Cidofovir (cCDV) | 2.10 | >1000 | >475 |
| 1-O-hexadecylpropanediol-3-CDV | 4 x 10⁻⁷ | 10 | 25 x 10⁶ |
| 1-O-hexadecylpropanediol-3-cCDV | 0.030 | 320 | 10,667 |
| 1-O-octadecylpropanediol-3-CDV | 0.003 | 32 | 10,667 |
| 1-O-octadecylpropanediol-3-cCDV | 0.330 | 320 | 970 |
| 1-O-octadecylpropanediol-2-CDV | 0.002 | 210 | 105,000 |
| 1-O-octadecylpropanediol-2-cCDV | 0.008 | 320 | 40,000 |

| | | | |
|---|---|---|---|
| Abbreviations as in Table 2. EC₅₀ - 50% effective concentration; CC₅₀ - 50% cytotoxic concentration; selectivity index - CC₅₀/EC₅₀. EC₅₀ values are the average of two experiments with the exception of HDP-CDV which is a single determination in duplicate. | | | |

### Example 11: Metabolic Stability Evaluation

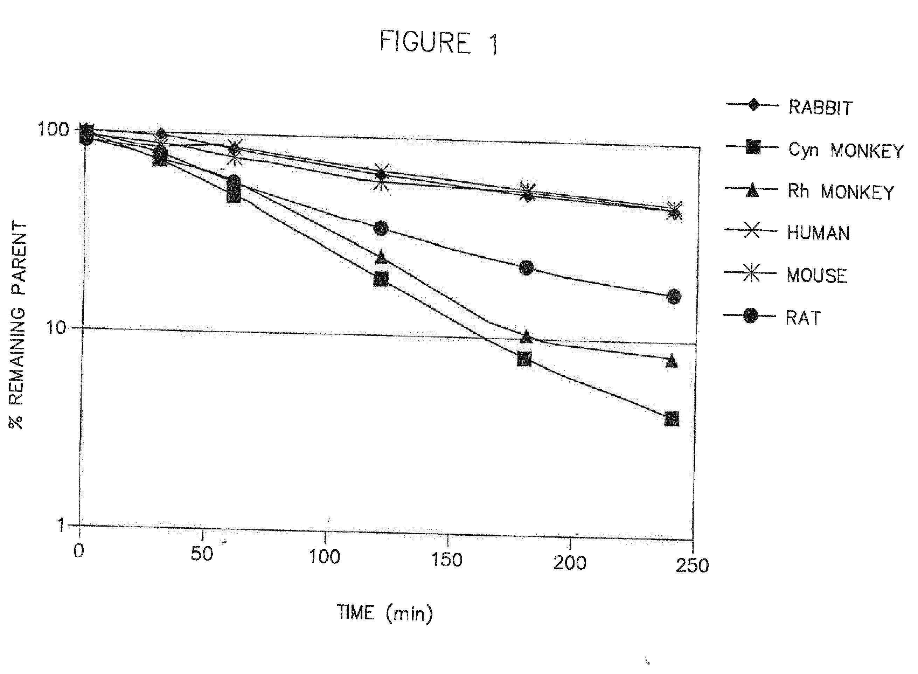

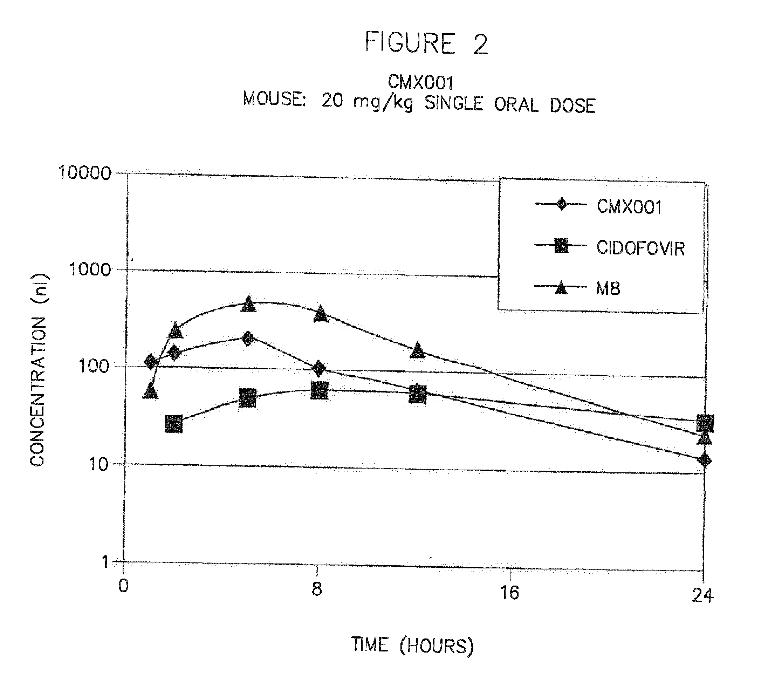

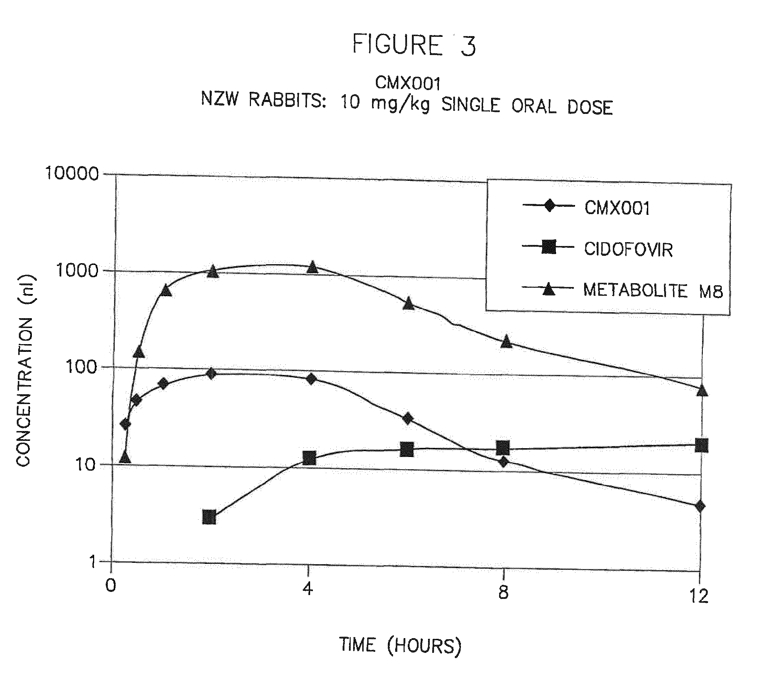

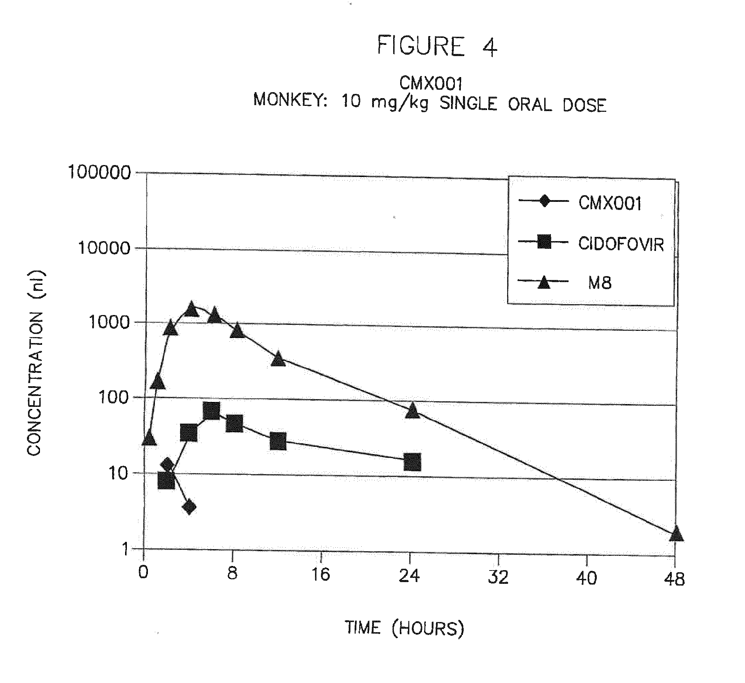

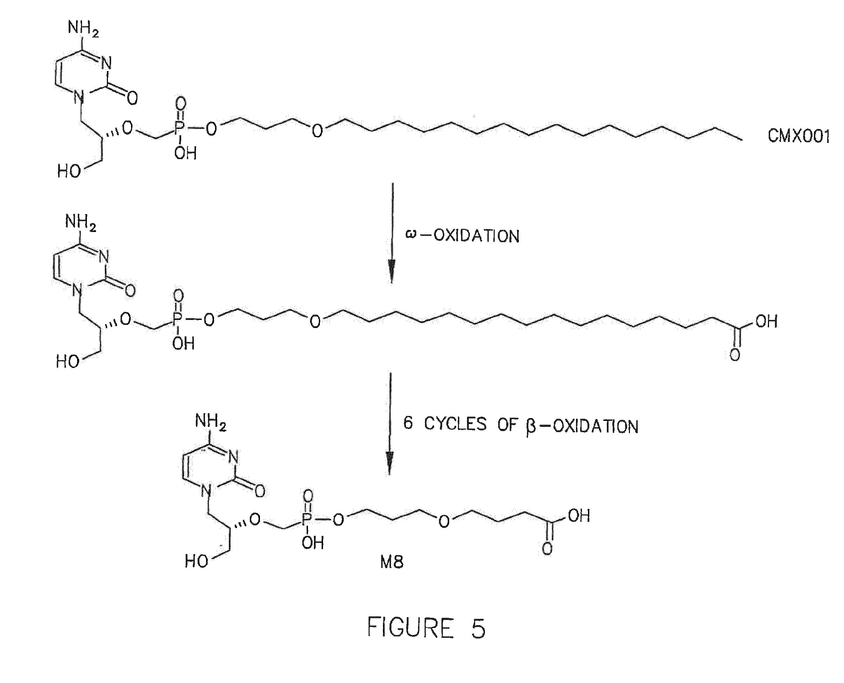

The metabolic stability of HDP-cidofovir (1 µM) in cryo-preserved primary hepatocytes was examined in rabbit, Cyn monkey, Rh monkey, human, mouse and rat. The results are shown in Figure 1, which shows the decrease in HDP-cidofovir over time. Additionally, the serum concentration of HDP-cidofovir, cidofovir released from HDP-cidofovir, and the metabolite M-8, which is an inactive metabolite of HDP-cidofovir, in mouse, NZW rabbits, and monkey was evaluated, and the results are shown in Figures 2, 3 and 4 after a single oral dose. Figure 5 shows a possible mechanism for the formation of M-8 by oxidation of HDP-cidofovir. In a quantitative whole body autoradiograph of a mouse 4 hours after a 5mg/kg oral dose of [2- 14C] HDP-cidofovir (hexadecyloxypropyl-cidofovir), the drug was identified as being distributed primarily in the GI tract as well as organs including the lung, heart, liver, and kidney.

### Example 12

### Titer Study in Monkeys

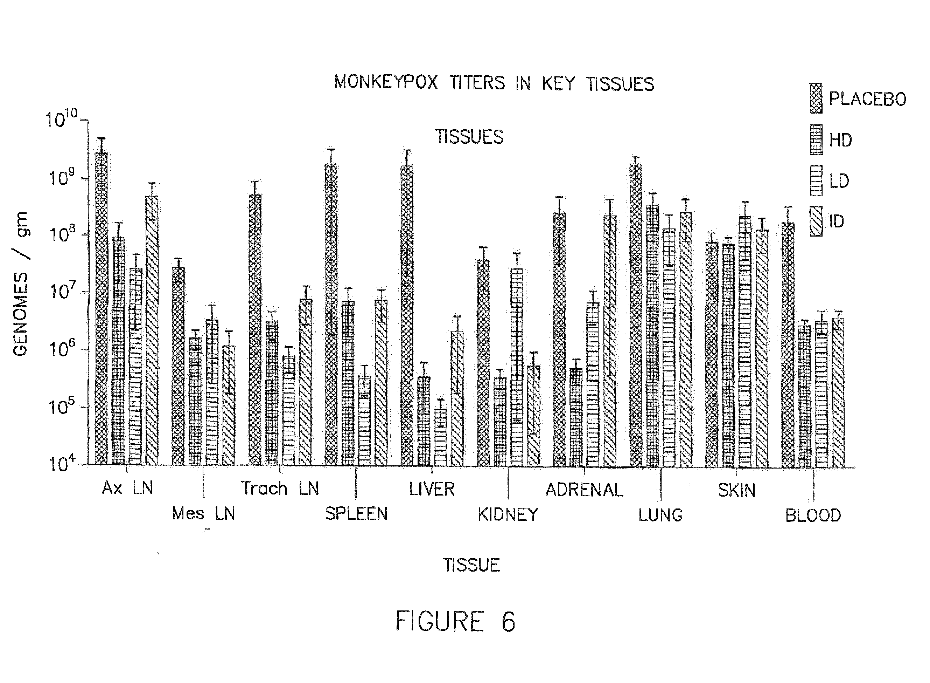

Sixteen Cynomolgus Macaques were tested with 4 treatment groups of 4 animals each. The animals were inoculated with 5 x 10⁷ PFU IV, Monkeypox virus, Zaire 79 strain. The animals were given placebos, or different dosage levels (TD: 35, 30, 20, 0 mg/kg), show in Figure 6 as HD, LD, ID and placebo respectively. Figure 6 shows the viral load of monkeypox titers in different types of tissue after drug administration.

### EMBODIMENTS

1. A method of treatment of a viral infection, the method comprising administering a lipid containing prodrug of an antiviral compound or a salt, ester or prodrug thereof, in combination or alternation with a bioavailability enhancer to a host in need thereof in an effective amount for treatment of the viral infection.
2. The method of embodiment 1, wherein the antiviral compound is a nucleoside.
3. The method of embodiment 1, wherein the antiviral compound is an anti-orthopox drug.
4. The method of embodiment 1, wherein the antiviral compound is an active against HIV, hepatitis B, or hepatitis C.
5. The method of embodiment 1 , wherein the bioavailability enhancer is an inhibitor or substrate of a cytochrome P450 enzyme; an imidazole; a macrolide; a calcium channel blocker; or a steroid.
6. The method of embodiment 1, wherein the bioavailability enhancer is an inhibitor of cytochrome P450 3A (CYP3A) or of P-glycoprotein-mediated membrane transport.
7. The method of embodiment 1, wherein the lipid containing prodrug of an antiviral compound or a salt, ester or prodrug thereof, and the bioavailability enhancer are administered in a pharmaceutically acceptable carrier in combination or alternation.
8. The method of embodiment 7, wherein the lipid containing prodrug of an antiviral compound or a salt, ester or prodrug thereof, and the bioavailability enhancer are administered by the same or different route selected from oral, topical or parenteral administration.
9. The method of embodiment 1, wherein the antiviral compound is cidofovir or cyclic cidofovir.
10. The method of embodiment 1, wherein the antiviral compound is cidofovir, adefovir, cyclic cidofovir or tenofovir, optionally covalently linked to an alkylpropanediol. 1-S-alkylthioglycerol, alkoxyalkanol or alkylethanediol.
11. The method of embodiment 9, wherein the antiviral compound has the structure: 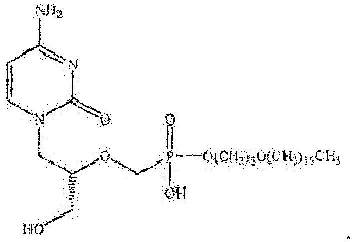
12. The method of embodiment 1, wherein the viral infection is variola major, variola minor, vaccinia, smallpox, cowpox, carnelpox, mousepox, rabbitpox, or monkeypox.
13. A use of an effective amount of a lipid containing prodrug of an antiviral compound or a salt, ester or prodrug thereof, in combination or alternation with a bioavailability enhancer in the manufacture of a medicament for treatment of the viral infection.
14. The use of embodiment 13, wherein the antiviral compound is a nucleoside.
15. The use of embodiment 13, wherein the antiviral compound is an anti-orthopox drug.
16. The use of embodiment 13, wherein the antiviral compound is an active against HIV, hepatitis B. or hepatitis C.
17. The use of embodiment 13, wherein the bioavailability enhancer is an inhibitor or substrate of a cytochrome P450 enzyme; an imidazole; a macrolide; a calcium channel blocker; or a steroid.
18. The use of embodiment 13, wherein the bioavailability enhancer is an inhibitor of cytochrome P450 3A (CYP3A) or of P-glycoprotein-mediated membrane transport.
19. The use of embodiment 13, wherein the antiviral compound is cidofovir or cyclic cidofovir.
20. The use of embodiment 13, wherein the antiviral compound is cidofovir, adefovir, cyclic cidofovir or tenofovir, optionally covalently linked to an alkylpropanediol, 1 -S-alkylthioglycerol, alkoxyalkanol or alkylethanediol.
21. The use of embodiment 19, wherein the antiviral compound has the structure: 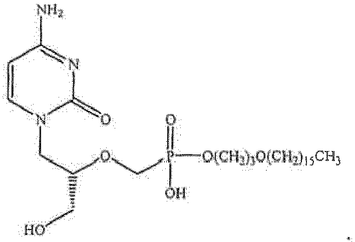
22. The use of embodiment 13, wherein the viral infection is variola major, variola minor, vaccinia, smallpox, cowpox, camelpox, mousepox, rabbitpox, or monkeypox.

## Claims

1. A compound for use in the treatment of a viral infection having the structure: 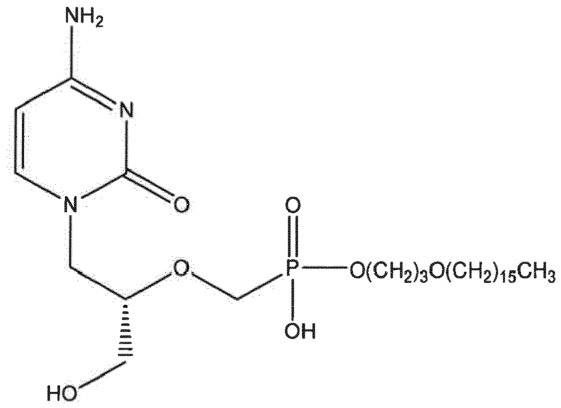 or a pharmaceutically acceptable salt thereof, in combination or alternation with an immunosuppressant;
wherein the viral infection is an orthopox, HIV, hepatitis B, hepatitis C, variola major, variola minor, vaccinia, smallpox, cowpox, camelpox, mousepox, rabbitpox, cytomegalovirus, herpes simplex virus type 1, herpes simplex virus type 2, papilloma virus, or monkeypox infection,
wherein the compound is administered at a dose of from about 1 mg to about 500 mg.

2. The compound of claim 1 for use according to claim 1, wherein the immunosuppressant is cyclosporine.

3. The compound of claim 1 for use according to claim 1, wherein the immunosuppressant is FK-506.

4. The compound of claim 1 for use according to claim 1, wherein the immunosuppressant is rapamycin.

5. The compound of claim 1 for use according to claim 1, wherein the compound and the immunosuppressant are administered in a pharmaceutically acceptable carrier.

6. The compound of claim 1 for use according to claim 1, wherein the compound and the immunosuppressant are administered by the same or different route selected from oral, topical or parenteral administration.

7. The compound of claim 1 for use according to claim 1, wherein the compound is administered in combination with the immunosuppressant.

8. The compound of claim 1 for use according to claim 1, wherein the compound is administered sequentially with the immunosuppressant.

9. The compound of claim 1 for use according to claim 1, wherein the compound is administered prior to or subsequent to the immunosuppressant.

10. The compound of claim 1 for use according to claim 1, wherein the compound is administered serially with the immunosuppressant.

11. The compound of claim 1 for use according to claim 1, wherein the compound is administered orally.

12. The compound of claim 1 for use according to claim 1, wherein the compound is administered at a dose of from about 10 mg to about 500 mg.

13. A compound for use in the treatment of a viral infection having the structure: 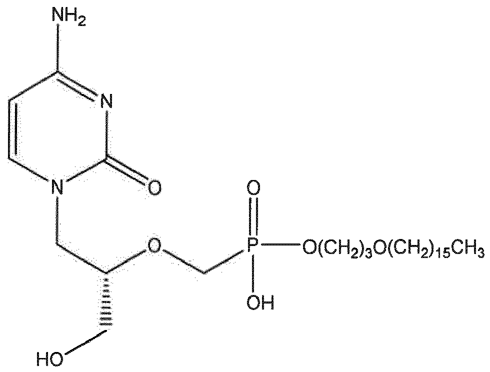 or a pharmaceutically acceptable salt thereof, in combination or alternation with an immunosuppressant;
wherein the viral infection is an influenza, pestivirus, classic swine fever virus, Border disease virus of sheep, flavivirus, yellow fever virus, West Nile virus, Japanese encephalitis virus, Varicella zoster virus, human herpes virus 6, Epstein-Barr virus, Herpes type 6, Herpes type 8, molluscum contagiosum, SIV, HIV-1, HIV-2, adenovirus or ebola virus infection;
wherein the compound is administered at a dose of from about 1 mg to about 500 mg.

14. The compound of claim 13 for use according to claim 13, wherein the viral infection is a flavivirus infection selected from the group consisting of: Absettarov, Alfuy, Apoi, Aroa, Bagaza, Banzi, Bouboui, Bussuquara, Cacipacore, Carey Island, Dakar bat, Dengue 1, Dengue 2, Dengue 3, Dengue 4, Edge Hill, Entebbe bat, Gadgets Gully, Hanzalova, Hypr, Ilheus, Israel turkey meningoencephalitis, Japanese encephalitis, Jugra, Jutiapa, Kadam, Karshi, Kedougou, Kokobera, Koutango, Kumlinge, Kunjin, Kyasanur Forest disease, Langat, Louping ill, Meaban, Modoc, Montana myotis leukoencephalitis, Murray valley encephalitis, Naranjal, Negishi, Ntaya, Omsk hemorrhagic fever, Phnom-Penh bat, Powassan, Rio Bravo, Rocio, Royal Farm, Russian spring-summer encephalitis, Saboya, St. Louis encephalitis, Sal Vieja, San Perlita, Saumarez Reef, Sepik, Sokuluk, Spondweni, Stratford, Tembusu, Tyuleniy, Uganda S, Usutu, Wesselsbron, West Nile, Yaounde, Yellow fever, and Zika.
